# EUROPEAN PATENT APPLICATION

(11) **EP 3 575 402 A1**
(43) Date of publication of application: **04.12.2019**
(21) Application number: 18175623.0
(22) Date of filing: 01.06.2018
(51) Int. Cl.: C12N 15/62, C12N 9/22, C12N 15/82

(54) **GENE TARGETING**

(71) Applicant: Algentech SAS, 91058 Evry Cedex (FR)
(72) Inventor: The designation of the inventor has not yet been filed
(74) Representative: Eyre, David Edward

(57) **Abstract**

Methods, reagents and compositions for providing more accurate and reliable genetic modification are provided. In particular a nucleic acid encoding a fusion protein comprising an endonuclease domain and a binding domain for an origin of replication is described. Also provided are methods, reagents and compositions for in vivo genetic modification of the genome of a non-animal cell or organism. Furthermore, the present application relates to uses of the said methods, reagents and compositions for introducing desirable traits to non-animal organisms or ameliorating or removing non-desirable traits in these organisms including in the treatment of disease.

SEQUENCE LISTING

<110>  Algentech SAS

<120>  GENE TARGETING

<130>  P40363EP

<160>  25

<170>  PatentIn version 3.5

<210>  1
<211>  1424
<212>  DNA
<213>  Cytomegalovirus (CMV)

<400>  1
ggaattccaa tcccacaaaa atctgagctt aacagcacag ttgctcctct cagagcagaa      60

tcgggtattc aacaccctca tatcaactac tacgttgtgt ataacggtcc acatgccggt     120

atatacgatg actggggttg tacaaaggcg gcaacaaacg gcgttcccgg agttgcacac     180

aagaaatttg ccactattac agaggcaaga gcagcagctg acgcgtacac aacaagtcag     240

caaacagaca ggttgaactt catccccaaa ggagaagctc aactcaagcc caagagcttt     300

gctaaggccc taacaagccc accaaagcaa aaagcccact ggctcacgct aggaaccaaa     360

aggcccagca gtgatccagc cccaaaagag atctcctttg ccccggagat tacaatggac     420

gatttcctct atctttacga tctaggaagg aagttcgaag gtgaagtaga cgacactatg     480

ttcaccactg ataatgagaa ggttagcctc ttcaatttca gaaagaatgc tgacccacag     540

atggttagag aggcctacgc agcaggtctc atcaagacga tctacccgag taacaatctc     600

caggagatca aataccttcc caagaaggtt aaagatgcag tcaaaagatt caggactaat     660

tgcatcaaga acacagagaa agacatattt ctcaagatca gaagtactat tccagtatgg     720

acgattcaag gcttgcttca taaaccaagg caagtaatag agattggagt ctctaaaaag     780

gtagttccta ctgaatctaa ggccatgcat ggagtctaag attcaaatcg aggatctaac     840

agaactcgcc gtgaagactg gcgaacagtt catacagagt cttttacgac tcaatgacaa     900

gaagaaaatc ttcgtcaaca tggtggagca cgacactctg gtctactcca aaaatgtcaa     960

agatacagtc tcagaagacc aaagggctat tgagactttt caacaaagga taatttcggg    1020

aaacctcctc ggattccatt gcccagctat ctgtcacttc atcgaaagga cagtagaaaa    1080

ggaaggtggc tcctacaaat gccatcattg cgataaagga aaggctatca ttcaagatct    1140

ctctgccgac agtggtccca aagatggacc cccacccacg aggagcatcg tggaaaaaga    1200

agacgttcca accacgtctt caaagcaagt ggattgatgt gacatctcca ctgacgtaag    1260

ggatgacgca caatcccact atccttcgca agacccttcc tctatataag gaagttcatt    1320

tcatttggag aggacacgct cgagtataag agctcatttt tacaacaatt accaacaaca    1380

acaaacaaca aacaacatta caattacatt tacaattatc gata 1424

<210> 2
<211> 1807
<212> DNA
<213> Escherichia coli

<400> 2
atgttacgtc ctgtagaaac cccaacccgt gaaatcaaaa aactcgacgg cctgtgggca 60

ttcagtctgg atcgcgaaaa ctgtggaatt gatcagcgtt ggtgggaaag cgcgttacaa 120

gaaagccggg caattgctgt gccaggcagt tttaacgatc agttcgccga tgcagatatt 180

cgtaattatg cgggcaacgt ctggtatcag cgcgaagtct ttataccgaa aggttgggca 240

ggccagcgta tcgtgctgcg tttcgatgcg gtcactcatt acggcaaagt gtgggtcaat 300

aatcaggaag tgatggagca tcagggcggc tatacgccat ttgaagccga tgtcacgccg 360

tatgttattg ccgggaaaag tacgtatcac cgtttgtgtg aacaacgaac tgaactggca 420

gactatcccg ccgggaatgg tgattaccga cgaaaacggc aagaaaaagc agtcttactt 480

ccatgatttc tttaactatg ccgcgatcca tcgcagcgta atgctctaca ccacgccgaa 540

cacctgggtg gacgatatca ccgtggtgac gcatgtcgcg caagactgta accacgcgtc 600

tgttgactgg caggtggtgg ccaatggtga tgtcagcgtt gaactgcgtg atgcggatca 660

acaggtggtt gcaactggac aaggcactag cgggactttg caagtggtga atccgcacct 720

ctggcaaccg ggtgaaggtt atctctatga actgtgcgtc acagccaaaa gccagacaga 780

gtgtgatatc tacccgcttc gcgtcggcat ccggtcagtg gcagtgaagg gcgaacagtt 840

cctgattaac cacaaaccgt tctactttac tggctttggt cgtcatgaag atgcggactt 900

acgtggcaaa ggattcgata acgtgctgat ggtgcacgac cacgcattaa tggactggat 960

tggggccaac tcctaccgta cctcgcatta cccttacgct gaagagatgc tcgactgggc 1020

agatgaacat ggcatcgtgg tgattgatga aactgctgct gtcggcttta acctctcttt 1080

aggcattggt ttcgaagcgg gcaacaagcc gaaagaactg tacagcgaag aggcagtcaa 1140

cggggaaact cagcaagcgc acttacaggc gattaaagag ctgatagcgc gtgacaaaaa 1200

ccacccaagc gtggtgatgt ggagtattgc caacgaaccg gatacccgtc cgcaagtgca 1260

cgggaatatt tcgccactgg cggaagcaac gcgtaaactc gacccgacgc gtccgatcac 1320

ctgcgtcaat gtaatgttct gcgacgctca caccgatacc atcagcgatc tctttgatgt 1380

gctgtgcctg aaccgttatt acggatggta tgtccaaagc ggcgatttgg aaacggcaga 1440

gaaggtactg gaaaaagaac ttctggcctg gcaggagaaa ctgcatcagc cgattatcat 1500

caccgaatac ggcgtggata cgttagccgg gctgcactca atgtacaccg acatgtggag 1560

tgaagagtat cagtgtgcat ggctggatat gtatcaccgc gtctttgatc gcgtcagcgc 1620

cgtcgtcggt gaacaggtat ggaatttcgc cgattttgcg acctcgcaag gcatattgcg 1680

cgttggcggt aacaagaaag ggatcttcac tcgcgaccgc aaaccgaagt cggcggcttt 1740

tctgctgcaa aaacgctgga ctggcatgaa cttcggtgaa aaaccgcagc agggaggcaa 1800

acaatga 1807

<210> 3
<211> 265
<212> DNA
<213> Agrobacterium tumefaciens

<400> 3
gtcgaagcag atcgttcaaa catttggcaa taaagtttct taagattgaa tcctgttgcc 60

ggtcttgcga tgattatcat ataatttctg ttgaattacg ttaagcatgt aataattaac 120

atgtaatgca tgacgttatt tatgagatgg gtttttatga ttagagtccc gcaattatac 180

atttaatacg cgatagaaaa caaaatatag cgcgcaaact aggataaatt atcgcgcgcg 240

gtgtcatcta tgttactaga tcgac 265

<210> 4
<211> 1505
<212> DNA
<213> Escherichia coli

<400> 4
cgtcctgtag aaaccccaac ccgtgaaatc aaaaaactcg acggcctgtg ggcattcagt 60

ctggatcgcg aaaactgtgg aattgatcag cgttggtggg aaagcgcgtt acaagaaagc 120

cgggcaattg ctgtgccagg cagttttaac gatcagttcg ccgatgcaga tattcgtaat 180

tatgcgggca acgtctggta tcagcgcgaa gtctttatac cgaaaggttg ggcaggccag 240

cgtatcgtgc tgcgtttcga tgcggtcact cattacggca aagtgtgggt caataatcag 300

gaagtgatgg agcatcaggg cggctatacg ccatttgaag ccgatgtcac gccgtatgtt 360

attgccggga aaagtgtacg tatcactgtt tgtgtgaaca acgaactgaa ctggcagact 420

atcccgccgg gaatggtgat taccgacgaa aacggcaaga aaaagcagtc ttacttccat 480

gatttcttta actatgccgg aatccatcgc agcgtaatgc tctacaccac gccgaacacc 540

tgggtggacg atatcaccgt ggtgacgcat gtcgcgcaag actgtaacca cgcgtctgtt 600

gactggcagg tggtggccaa tggtgatgtc agcgttgaac tgcgtgatgc ggatcaacag 660

gtggttgcaa ctggacaagg cactagcggg actttgcaag tggtgaatcc gcacctctgg 720

caaccgggtg aaggttatct ctatgaactg tgcgtcacag ccaaaagcca gacagagtgt 780

gatatctacc cgcttcgcgt cggcatccgg tcagtggcag tgaagggcga acagttcctg 840

attaaccaca aaccgttcta ctttactggc tttggtcgtc atgaagatgc ggacttgcgt 900

ggcaaaggat tcgataacgt gctgatggtg cacgaccacg cattaatgga ctggattggg 960

gccaactcct accgtacctc gcattaccct tacgctgaag agatgctcga ctgggcagat 1020

gaacatggca tcgtggtgat tgatgaaact gctgctgtcg gctttaacct ctctttaggc 1080

attggtttcg aagcgggcaa caagccgaaa gaactgtaca gcgaagaggc agtcaacggg 1140

gaaactcagc aagcgcactt acaggcgatt aaagagctga tagcgcgtga caaaaaccac 1200

ccaagcgtgg tgatgtggag tattgccaac gaaccggata cccgtccgca aggtgcacgg 1260

gaatatttcg cgccactggc ggaagcaacg cgtaaactcg acccgacgcg tccgatcacc 1320

tgcgtcaatg taatgttctg cgacgctcac accgatacca tcagcgatct ctttgatgtg 1380

ctgtgcctga accgttatta cggatggtat gtccaaagcg gcgatttgga aacggcagag 1440

aaggtactgg aaaaagaact tctggcctgg caggagaaac tgcatcagcc gattatcatc 1500

accga 1505

<210> 5
<211> 2676
<212> DNA
<213> Nicotiana tabacum

<400> 5
tgctgccgga tgcaatcagt gcaaggtgcc cttctccttt cccagttcaa tgtctccacc 60

ccactcccac ccccagtata aaaattcagg ggggaaaaag agaagaaaaa tatcaaaatt 120

taaggactag ttaaagttca gttttggcat tttttctgcc taagtgcaat attatcaaag 180

gtgaaaagcg caaaaaagct ctaaagtcta ttggggcttt aagcgcaaag cgcaaataaa 240

gcatgggctt taatgcagaa aggctcaaat gaaaatgaaa agaaaaagaa aactacaaat 300

atgtatgtat agtccaagac taatatctat aagcatgaat accaaatata tggacaaaga 360

cattgaagaa actttattat aaagtgaaat atcaattgtt tagcatcgcc tcttcaggat 420

tacgcttatt ggcaaggaaa agtatgcttt tagagccttg atgacaacgc tgaagcgccc 480

gctaagcgag gcaaagcgct caacatgttt tgagcctcgc ttcagggctt aagcgttatc 540

aaagtgtgtt ctttggtgcc cttgttcaag cagttctcta gtatttttac cttggctcca 600

ccccactccc accccctgta taaacgttta gggggggaaa agaaagagaa gaaaaattat 660

caaaagttaa gaaagctaag ttttggcttt tttctgctag gtgtgttctt tggtgctctt 720

gttcaagcag ttctctagta tttttacctt ggaaatgcta ataagttgtc gccgtgtttc 780

cttagattgg gaagcaaatt aaaactatca tggaagcttc ttagcattac taagtcagaa 840

aaagcaggat atcgcttgac atacgagaca ccagaaggag tagtttctct tcaaagtcga 900

agcattgtca tgactgtgcc atcctatgta gcaagcaaca tattacgtcc tctttcggtg 960

tgtttccatt actgtttgct ccaaagtcgt tattgctatt gttcatttgg cactctaata 1020

tagaaacttg gataaaaaag gggatccttt aatttttctt tttcatttgg actaggaaaa 1080

gcggctcaaa cacatgtata acagtcatat actgtgtata atatgtgtat gcaatataat 1140

gtgcagagaa agacttatcg gagagaagag aattaaaaaa aagaacattt gtgtcacacg 1200

gagaattaac atgtacatgc ttaatgaggt caagagcgca accgtatccc acccctcatg 1260

aaagattgaa cctgtatctc atgttattgt gagctttttt catatataaa tttatgagca 1320

attttcttta aatgctttga ccttggattt ttcgaaggtt actacttctg ataattgctt 1380

tgcaagaaat gcggctcact taaaatggga aacgtttatc tataggttgt ggttcagctt 1440

cttctgtttc ttgctaaagc aagatgtctc ctaaacttat attaaactca ggttgccgca 1500

gcagatgcac tttcaaattt ctactatccc ccagttggag cagtcacaat ttcatatcct 1560

caagaagcta ttcgtgatga acgtctggtt gatggtgaac taaagggatt tgggcagttg 1620

catccacgta cacagggagt ggaaacacta ggtagctttc tgtttgaagg attcactcta 1680

attttaactt caagaatcca tattgaagat ggaaaacttt tattacgaca attgcaagat 1740

gcaactcact gaagtagcat tcctctattg catctgcagg aataatatat agcgtatcag 1800

tcttccctga ccatcattac ctagaaattt tgacagtacc atgcaaatcg aagatgaatg 1860

agttcagaac aataacatca ggatttgatt catcttaaat ataatacttg cgtttcatat 1920

gcaggaacga tatatagttc atcactcttc cctaaccgtg ccccaaaagg tcgggtgcta 1980

ctcttgaaca tgattgcagg agcaaaaaat ccggaaattt tgtctaaggt aaggcattta 2040

aattgagcgc aacttcaagt cctgccggta ttgatttatc ataatgaata ttcatgggct 2100

ggccagttgg ggcatcgctt gaaggttcct atggtaacaa acagaaaact gagaatgacc 2160

ttatcccctg atctcttttg gttgcaaata cgttaacctt gcagtgatga ggagaatgtt 2220

ttcagtgtta ttttctctag attaatggcg tagttggttt ggattacaaa accgtttagg 2280

cttatatgtg attagagtct ccaggttttt gatctagcag taagagcaaa atgtgtgatg 2340

ttttaaaaaa acagtcaagt gattacaaat actcagaata gtttcaccat aaagaactaa 2400

agagctaaga agattgtggt ttctctttcc tctatgttcg actgcatttt gtgggttttc 2460

catagcatga ctttctttta tggtacttgt acgaaagcat ttcgctgtta ttctttatgc 2520

ttcagggcct agaataaatt gctttgagat ttgatccctc attcttcttg cagacggaga 2580

gccaacttgt ggaagtagtt gatcgtgacc tcagaaaaat gcttatagaa cccaaagcac 2640

aagatcccct tgttgtgggt gtgcgagtat ggccac 2676

<210> 6
<211> 204
<212> DNA
<213> Beet curly top virus

<400> 6

tcctgtactc cgatgacgtg gcttagcata ttaacatatc tattggagta ttggagtatt 60

atatatatta gtacaacttt cataagggcc atccgttata atattaccgg atggcccgaa 120

aaaaatgggc acccaatcaa aacgtgacac gtggaagggg actgttgaat gatgtgacgt 180

ttttgagcgg gaaacttcct gaag 204

<210> 7
<211> 1649
<212> DNA
<213> Beet curly top virus

<400> 7
atgcctccta ctaaaagatt tcgtattcaa gcaaaaaaca tatttcttac atatcctcag 60

tgttctcttt caaaagaaga agctcttgag caaattcaaa gaatacaact ttcatctaat 120

aaaaaatata ttaaaattgc cagagagcta cacgaagatg ggcaacctca tctccacgtc 180

ctgcttcaac tcgaaggaaa agttcagatc acaaatatca gattattcga cctggtatcc 240

ccaaccaggt cagcacattt ccatccaaac attcagagag ctaaatccag ctccgacgtc 300

aagtcctacg tagacaagga cggagacaca attgaatggg gagaattcca gatcgacggt 360

agaagtgcta gaggaggtca acagacagct aacgactcat atgccaaggc gttaaacgca 420

acttctcttg accaagcact tcaaatattg aaggaagaac aaccaaagga ttacttcctt 480

caacatcaca atcttttgaa caatgctcaa aagatatttc agaggccacc tgatccatgg 540

actccactat ttcctctgtc ctcattcaca aacgttcctg aggaaatgca agaatgggct 600

gatgcatatt tcggggttga tgccgctgcg cggcctttaa gatataatag tatcatagta 660

gagggtgatt caagaacagg gaagactatg tgggctagat ctttaggggc ccacaattac 720

atcacagggc acttagattt tagccctaga acgtattatg atgaagtgga atacaacgtc 780

attgatgacg tagatcccac ttacttaaag atgaaacact ggaaacacct tattggagca 840

caaaaggagt ggcagacaaa cttaaagtat ggaaaaccac gtgtcattaa aggtggtatc 900

ccctgcatta tattatgcaa tccaggacct gagagctcat accaacaatt tcttgaaaaa 960

ccagaaaatg aagcccttaa gtcctggaca ttacataatt caaccttctg caaactccaa 1020

ggtccgctct ttaataacca agcagcagca tcctcgcaag gtgactctac cctgtaactg 1080

ccacttcaca atacaccatg aatgtaatag aggattttcg cacaggggaa cctattactc 1140

tccatcaggc aacaaattcc gtcgaattcg agaatgtacc gaatccactg tatatgaaac 1200

tcctatggtt cgagagatac gggccaatct atcaactgaa gatacaaatc agattcaact 1260

acaacctccg gagagcgttg aatcttcaca agtgctggat agagctgacg ataactggat 1320

cgaacaggat attgactgga ccccgtttct tgaaggtctt gaaaaagaga ctagagatat 1380

acttggataa tctaggttta atttgtatta ataatgtaat tagaggttta aatcatgtcc 1440

tgtatgaaga atttactttt gtatcaagtg taattcagaa ccagagtgtt gcaatgaact 1500

tgtactaatt tcattattaa taataaatat tattaataaa aatagcatct acaattgcca 1560

aataatgtgg catacatatt agtattatcc gtattatcat taacaacaac atagagtaaa 1620

gcattctcct tcacgtcttc atacttccc 1649

<210> 8
<211> 4140
<212> DNA
<213> Streptococcus pyogenes

<400> 8
atggacaaga agtacagcat cggcctggac atcggcacga actcggtggg ctgggcggtg 60

atcacggacg agtacaaggt gccctccaag aagttcaagg tgctgggcaa caccgaccgc 120

cactcgatca agaagaacct gatcggcgcc ctgctgttcg actccggcga gaccgccgag 180

gcgacgcgcc tgaagcgcac cgcgcgtcgc cgctacacgc gtcgcaagaa ccgcatctgc 240

tacctgcagg agatcttcag caacgagatg gccaaggtgg acgactcgtt cttccaccgc 300

ctggaggagt ccttcctggt ggaggaagac aagaagcacg agcgccaccc catcttcggc 360

aacatcgtgg acgaggtggc ctaccacgag aagtacccga cgatctacca cctgcgcaag 420

aagctggtgg acagcaccga caaggcggac ctgcgcctga tctacctggc cctggcgcac 480

atgatcaagt tccgcggcca cttcctgatc gagggcgacc tgaaccccga caactcggac 540

gtggacaagc tgttcatcca gctggtgcag acctacaacc agctgttcga ggagaacccg 600

atcaacgcct ccggcgtgga cgccaaggcg atcctgagcg cgcgcctgtc caagagccgt 660

cgcctggaga acctgatcgc ccagctgccc ggcgagaaga agaacggcct gttcggcaac 720

ctgatcgcgc tgtcgctggg cctgacgccg aacttcaagt ccaacttcga cctggccgag 780

gacgcgaagc tgcagctgag caaggacacc tacgacgacg acctggacaa cctgctggcc 840

cagatcggcg accagtacgc ggacctgttc ctggccgcga agaacctgtc ggacgccatc 900

ctgctgtccg acatcctgcg cgtgaacacc gagatcacga aggcccccct gtcggcgtcc 960

atgatcaagc gctacgacga gcaccaccag gacctgaccc tgctgaaggc gctggtgcgc 1020

cagcagctgc cggagaagta caaggagatc ttcttcgacc agagcaagaa cggctacgcc 1080

ggctacatcg acggcggcgc gtcgcaagag gagttctaca agttcatcaa gcccatcctg 1140

gagaagatgg acggcacgga ggagctgctg gtgaagctga accgcgagga cctgctgcgc 1200

aagcagcgca ccttcgacaa cggcagcatc ccccaccaga tccacctggg cgagctgcac 1260

gccatcctgc gtcgccaaga ggacttctac ccgttcctga aggacaaccg cgagaagatc 1320

gagaagatcc tgacgttccg catcccctac tacgtgggcc cgctggcccg cggcaacagc 1380

cgcttcgcgt ggatgacccg caagtcggag gagaccatca cgccctggaa cttcgaggaa 1440

gtggtggaca agggcgccag cgcgcagtcg ttcatcgagc gcatgaccaa cttcgacaag 1500

aacctgccca acgagaaggt gctgccgaag cactccctgc tgtacgagta cttcaccgtg 1560

tacaacgagc tgacgaaggt gaagtacgtg accgagggca tgcgcaagcc cgccttcctg 1620

agcggcgagc agaagaaggc gatcgtggac ctgctgttca agaccaaccg caaggtgacg 1680

gtgaagcagc tgaaagagga ctacttcaag aagatcgagt gcttcgacag cgtggagatc 1740

tcgggcgtgg aggaccgctt caacgccagc ctgggcacct accacgacct gctgaagatc 1800

atcaaggaca aggacttcct ggacaacgag gagaacgagg acatcctgga ggacatcgtg 1860

ctgaccctga cgctgttcga ggaccgcgag atgatcgagg agcgcctgaa gacgtacgcc 1920

cacctgttcg acgacaaggt gatgaagcag ctgaagcgtc gccgctacac cggctggggc 1980

cgcctgagcc gcaagctgat caacggcatc cgcgacaagc agtccggcaa gaccatcctg 2040

gacttcctga agagcgacgg cttcgcgaac cgcaacttca tgcagctgat ccacgacgac 2100

tcgctgacct tcaaagagga catccagaag gcccaggtgt cgggccaggg cgactccctg 2160

cacgagcaca tcgccaacct ggcgggctcc cccgcgatca agaagggcat cctgcagacc 2220

gtgaaggtgg tggacgagct ggtgaaggtg atgggccgcc acaagccgga gaacatcgtg 2280

atcgagatgg cccgcgagaa ccagaccacg cagaagggcc agaagaacag ccgcgagcgc 2340

atgaagcgca tcgaggaagg catcaaggag ctgggctcgc agatcctgaa ggagcacccc 2400

gtggagaaca cccagctgca gaacgagaag ctgtacctgt actacctgca gaacggccgc 2460

gacatgtacg tggaccagga gctggacatc aaccgcctgt ccgactacga cgtggaccac 2520

atcgtgcccc agagcttcct gaaggacgac tcgatcgaca acaaggtgct gacccgcagc 2580

gacaagaacc gcggcaagag cgacaacgtg ccgtcggagg aagtggtgaa gaagatgaag 2640

aactactggc gccagctgct gaacgccaag ctgatcacgc agcgcaagtt cgacaacctg 2700

accaaggccg agcgcggtgg cctgtcggag ctggacaagg cgggcttcat caagcgccag 2760

ctggtggaga cccgccagat cacgaagcac gtggcgcaga tcctggactc ccgcatgaac 2820

acgaagtacg acgagaacga caagctgatc cgcgaggtga aggtgatcac cctgaagtcc 2880

aagctggtca gcgacttccg caaggacttc cagttctaca aggtgcgcga gatcaacaac 2940

taccaccacg cccacgacgc gtacctgaac gccgtggtgg gcaccgcgct gatcaagaag 3000

taccccaagc tggagagcga gttcgtgtac ggcgactaca aggtgtacga cgtgcgcaag 3060

atgatcgcca agtcggagca ggagatcggc aaggccaccg cgaagtactt cttctactcc 3120

aacatcatga acttcttcaa gaccgagatc acgctggcca acggcgagat ccgcaagcgc 3180

ccgctgatcg agaccaacgg cgagacgggc gagatcgtgt gggacaaggg ccgcgacttc 3240

gcgaccgtgc gcaaggtgct gagcatgccc caggtgaaca tcgtgaagaa gaccgaggtg 3300

cagacgggcg gcttctccaa ggagagcatc ctgccgaagc gcaactcgga caagctgatc 3360

gcccgcaaga aggactggga ccccaagaag tacggcggct tcgactcccc gaccgtggcc 3420

tacagcgtgc tggtggtggc gaaggtggag aagggcaagt ccaagaagct gaagagcgtg 3480

aaggagctgc tgggcatcac catcatggag cgcagctcgt tcgagaagaa ccccatcgac 3540

ttcctggagg ccaagggcta caaagaggtg aagaaggacc tgatcatcaa gctgccgaag 3600

tactcgctgt tcgagctgga gaacggccgc aagcgcatgc tggcctccgc gggcgagctg 3660

cagaagggca acgagctggc cctgcccagc aagtacgtga acttcctgta cctggcgtcc 3720

cactacgaga agctgaaggg ctcgccggag gacaacgagc agaagcagct gttcgtggag 3780

cagcacaagc actacctgga cgagatcatc gagcagatct cggagttctc caagcgcgtg 3840

atcctggccg acgcgaacct ggacaaggtg ctgagcgcct acaacaagca ccgcgacaag 3900

cccatccgcg agcaggcgga gaacatcatc cacctgttca ccctgacgaa cctgggcgcc 3960

ccggccgcgt tcaagtactt cgacaccacg atcgaccgca agcgctacac ctccacgaaa 4020

gaggtgctgg acgcgaccct gatccaccag agcatcaccg gcctgtacga gacgcgcatc 4080

gacctgagcc agctgggcgg cgactcccgc gcggacccga agaagaagcg caaggtgtaa 4140

<210> 9
<211> 201
<212> DNA
<213> Arabidopsis thaliana

<400> 9
catcttcatt cttaagatat gaagataatc ttcaaaaggc ccctgggaat ctgaaagaag 60

agaagcaggc ccatttatat gggaaagaac aatagtattt cttatatagg cccatttaag 120

ttgaaaacaa tcttcaaaag tcccacatcg cttagataag aaaacgaagc tgagtttata 180

tacagctaga gtcgaagtag t 201

<210> 10
<211> 166
<212> DNA
<213> Artificial Sequence

<220>
<223> sgRNA2.0 with MS2 aptamer hairpins

<400> 10
cagttcgttg ttcacacaag ttttagagct aggccaacat gaggatcacc catgtctgca 60

gggcctagca agttaaaata aggctagtcc gttatcaact tggccaacat gaggatcacc 120

catgtctgca gggccaagtg gcaccgagtc ggtgcttttt tttttt 166

<210> 11
<211> 164
<212> DNA
<213> Artificial Sequence

<220>
<223>  sgRNA2.0-1 for tobacco PPOX1 gene

<400>  11
attactaagt cagaaaagtt ttagagctag gccaacatga ggatcaccca tgtctgcagg      60

gcctagcaag ttaaaataag gctagtccgt tatcaacttg gccaacatga ggatcaccca     120

tgtctgcagg gccaagtggc accgagtcgg tgcttttttt tttt                      164


<210>  12
<211>  167
<212>  DNA
<213>  Artificial Sequence

<220>
<223>  sgRNA2.0-2 for tobacco PPOX1 gene

<400>  12
tgctactctt gaactacatg gttttagagc taggccaaca tgaggatcac ccatgtctgc      60

agggcctagc aagttaaaat aaggctagtc cgttatcaac ttggccaaca tgaggatcac     120

ccatgtctgc agggccaagt ggcaccgagt cggtgctttt ttttttt                   167


<210>  13
<211>  406
<212>  DNA
<213>  Agrobacterium tumefaciens

<400>  13
gaattaacag aggtggatgg acagacccgt tcttacaccg gactgggcgc gggataggat      60

attcagattg ggatgggatt gagcttaaag ccggcgctga gaccatgctc aaggtaggca     120

atgtcctcag cgtcgagccc ggcatctatg tcgagggcat tggtggagcg cgcttcgggg     180

ataccgtgct tgtaactgag accggatatg aggccctcac tccgcttgat cttggcaaag     240

atatttgacg catttattag tatgtgttaa ttttcatttg cagtgcagta ttttctattc     300

gatctttatg taattcgtta caattaataa atattcaaat cagattattg actgtcattt     360

gtatcaaatc gtgtttaatg gatattttta ttataatatt gatgat                    406


<210>  14
<211>  6024
<212>  DNA
<213>  Artificial Sequence

<220>
<223>  cas9-B-rep fusion gene

<400>  14
atggacaaga agtacagcat cggcctggac atcggcacga actcggtggg ctgggcggtg      60

atcacggacg agtacaaggt gccctccaag aagttcaagg tgctgggcaa caccgaccgc     120

cactcgatca agaagaacct gatcggcgcc ctgctgttcg actccggcga gaccgccgag     180

gcgacgcgcc tgaagcgcac cgcgcgtcgc cgctacacgc gtcgcaagaa ccgcatctgc 240

tacctgcagg agatcttcag caacgagatg gccaaggtgg acgactcgtt cttccaccgc 300

ctggaggagt ccttcctggt ggaggaagac aagaagcacg agcgccaccc catcttcggc 360

aacatcgtgg acgaggtggc ctaccacgag aagtacccga cgatctacca cctgcgcaag 420

aagctggtgg acagcaccga caaggcggac ctgcgcctga tctacctggc cctggcgcac 480

atgatcaagt tccgcggcca cttcctgatc gagggcgacc tgaaccccga caactcggac 540

gtggacaagc tgttcatcca gctggtgcag acctacaacc agctgttcga ggagaacccg 600

atcaacgcct ccggcgtgga cgccaaggcg atcctgagcg cgcgcctgtc caagagccgt 660

cgcctggaga acctgatcgc ccagctgccc ggcgagaaga agaacggcct gttcggcaac 720

ctgatcgcgc tgtcgctggg cctgacgccg aacttcaagt ccaacttcga cctggccgag 780

gacgcgaagc tgcagctgag caaggacacc tacgacgacg acctggacaa cctgctggcc 840

cagatcggcg accagtacgc ggacctgttc ctggccgcga agaacctgtc ggacgccatc 900

ctgctgtccg acatcctgcg cgtgaacacc gagatcacga aggcccccct gtcggcgtcc 960

atgatcaagc gctacgacga gcaccaccag gacctgaccc tgctgaaggc gctggtgcgc 1020

cagcagctgc cggagaagta caaggagatc ttcttcgacc agagcaagaa cggctacgcc 1080

ggctacatcg acggcggcgc gtcgcaagag gagttctaca agttcatcaa gcccatcctg 1140

gagaagatgg acggcacgga ggagctgctg gtgaagctga accgcgagga cctgctgcgc 1200

aagcagcgca ccttcgacaa cggcagcatc ccccaccaga tccacctggg cgagctgcac 1260

gccatcctgc gtcgccaaga ggacttctac ccgttcctga aggacaaccg cgagaagatc 1320

gagaagatcc tgacgttccg catcccctac tacgtgggcc cgctggcccg cggcaacagc 1380

cgcttcgcgt ggatgacccg caagtcggag gagaccatca cgccctggaa cttcgaggaa 1440

gtggtggaca agggcgccag cgcgcagtcg ttcatcgagc gcatgaccaa cttcgacaag 1500

aacctgccca acgagaaggt gctgccgaag cactccctgc tgtacgagta cttcaccgtg 1560

tacaacgagc tgacgaaggt gaagtacgtg accgagggca tgcgcaagcc cgccttcctg 1620

agcggcgagc agaagaaggc gatcgtggac ctgctgttca agaccaaccg caaggtgacg 1680

gtgaagcagc tgaaagagga ctacttcaag aagatcgagt gcttcgacag cgtggagatc 1740

tcgggcgtgg aggaccgctt caacgccagc ctgggcacct accacgacct gctgaagatc 1800

atcaaggaca aggacttcct ggacaacgag gagaacgagg acatcctgga ggacatcgtg 1860

ctgaccctga cgctgttcga ggaccgcgag atgatcgagg agcgcctgaa gacgtacgcc 1920

cacctgttcg acgacaaggt gatgaagcag ctgaagcgtc gccgctacac cggctggggc 1980

cgcctgagcc gcaagctgat caacggcatc cgcgacaagc agtccggcaa gaccatcctg 2040

gacttcctga agagcgacgg cttcgcgaac cgcaacttca tgcagctgat ccacgacgac 2100

tcgctgacct tcaaagagga catccagaag gcccaggtgt cgggccaggg cgactccctg 2160

cacgagcaca tcgccaacct ggcgggctcc cccgcgatca agaagggcat cctgcagacc 2220

gtgaaggtgg tggacgagct ggtgaaggtg atgggccgcc acaagccgga gaacatcgtg 2280

atcgagatgg cccgcgagaa ccagaccacg cagaagggcc agaagaacag ccgcgagcgc 2340

atgaagcgca tcgaggaagg catcaaggag ctgggctcgc agatcctgaa ggagcacccc 2400

gtggagaaca cccagctgca gaacgagaag ctttacctgt actacctgca gaacggccgc 2460

gacatgtacg tggaccagga gctggacatc aaccgcctgt ccgactacga cgtggaccac 2520

atcgtgcccc agagcttcct gaaggacgac tcgatcgaca acaaggtgct gacccgcagc 2580

gacaagaacc gcggcaagag cgacaacgtg ccgtcggagg aagtggtgaa gaagatgaag 2640

aactactggc gccagctgct gaacgccaag ctgatcacgc agcgcaagtt cgacaacctg 2700

accaaggccg agcgcggtgg cctgtcggag ctggacaagg cgggcttcat caagcgccag 2760

ctggtggaga cccgccagat cacgaagcac gtggcgcaga tcctggactc ccgcatgaac 2820

acgaagtacg acgagaacga caagctgatc cgcgaggtga aggtgatcac cctgaagtcc 2880

aagctggtca gcgacttccg caaggacttc cagttctaca aggtgcgcga gatcaacaac 2940

taccaccacg cccacgacgc gtacctgaac gccgtggtgg gcaccgcgct gatcaagaag 3000

taccccaagc tggagagcga gttcgtgtac ggcgactaca aggtgtacga cgtgcgcaag 3060

atgatcgcca agtcggagca ggagatcggc aaggccaccg cgaagtactt cttctactcc 3120

aacatcatga acttcttcaa gaccgagatc acgctggcca acggcgagat ccgcaagcgc 3180

ccgctgatcg agaccaacgg cgagacgggc gagatcgtgt gggacaaggg ccgcgacttc 3240

gcgaccgtgc gcaaggtgct gagcatgccc caggtgaaca tcgtgaagaa gaccgaggtg 3300

cagacgggcg gcttctccaa ggagagcatc ctgccgaagc gcaactcgga caagctgatc 3360

gcccgcaaga aggactggga ccccaagaag tacggcggct tcgactcccc gaccgtggcc 3420

tacagcgtgc tggtggtggc gaaggtggag aagggcaagt ccaagaagct gaagagcgtg 3480

aaggagctgc tgggcatcac catcatggag cgcagctcgt tcgagaagaa ccccatcgac 3540

ttcctggagg ccaagggcta caaagaggtg aagaaggacc tgatcatcaa gctgccgaag 3600

tactcgctgt tcgagctgga gaacggccgc aagcgcatgc tggcctccgc gggcgagctg 3660

cagaagggca acgagctggc cctgcccagc aagtacgtga acttcctgta cctggcgtcc 3720

cactacgaga agctgaaggg ctcgccggag gacaacgagc agaagcagct gttcgtggag 3780

cagcacaagc actacctgga cgagatcatc gagcagatct cggagttctc caagcgcgtg 3840

atcctggccg acgcgaacct ggacaaggtg ctgagcgcct acaacaagca ccgcgacaag 3900

cccatccgcg agcaggcgga gaacatcatc cacctgttca ccctgacgaa cctgggcgcc 3960

ccggccgcgt tcaagtactt cgacaccacg atcgaccgca agcgctacac ctccacgaaa 4020

gaggtgctgg acgcgaccct gatccaccag agcatcaccg gcctgtacga gacgcgcatc 4080

gacctgagcc agctgggcgg cgactcccgc gcggacccga agaagaagcg caaggtgagc 4140

gctggaggag gtggaagcgg aggaggagga agcggaggag gaggtagcgg atccatgcct 4200

cctactaaaa gatttcgtat tcaagcaaaa aacatatttc ttacatatcc tcagtgttct 4260

ctttcaaaag aagaagctct tgagcaaatt caaagaatac aactttcatc taataaaaaa 4320

tatattaaaa ttgccagaga gctacacgaa gatgggcaac ctcatctcca cgtcctgctt 4380

caactcgaag gaaaagttca gatcacaaat atcagattat tcgacctggt atccccaacc 4440

aggtaatttt catctttgtt tggccttcca agtgcttttt ttgctgttta cgggtggaac 4500

ttcagtaaaa atgggatcaa aacatcatat ggcataaata aattttaaga atggcgaact 4560

cggggttacc gaatatggct tcctttttca gtgtttctta gtccattgta cttatgagat 4620

tgcaggtcag cacatttcca tccaaacatt cagagagcta aatccagctc cgacgtcaag 4680

tcctacgtag acaaggacgg agacacaatt gaatggggag aattccagat cgacggtaga 4740

agtgctagag gaggtcaaca gacagctaac gactcatatg ccaaggcgtt aaacgcaact 4800

tctcttgacc aagcacttca aatattgaag gaagaacaac caaaggatta cttccttcaa 4860

catcacaatc ttttgaacaa tgctcaaaag atatttcaga ggccacctga tccatggact 4920

ccactatttc ctctgtcctc attcacaaac gttcctgagg aaatgcaaga atgggctgat 4980

gcatatttcg gggttgatgc cgctgcgcgg cctttaagat ataatagtat catagtagag 5040

ggtgattcaa gaacagggaa gactatgtgg gctagatctt taggggccca caattacatc 5100

acagggcact tagattttag ccctagaacg tattatgatg aagtggaata caacgtcatt 5160

gatgacgtag atcccactta cttaaagatg aaacactgga aacaccttat tggagcacaa 5220

aaggagtggc agacaaactt aaagtatgga aaaccacgtg tcattaaagg tggtatcccc 5280

tgcattatat tatgcaatcc aggacctgag agcacatacc aacaatttct tgaaaaacca 5340

gaaaatgaag cccttaagtc ctggacatta cataattcaa ccttctgcaa actccaaggt 5400

ccgctcttta ataaccaagc agcagcatcc tcgcaaggtg actctaccct gtaactgcca 5460

cttcacaata caccatgaat gtaatagagg attttcgcac aggggaacct attactctcc 5520

atcaggcaac aaattccgtc gaattcgaga atgtaccgaa tccactgtat atgaaactcc 5580

tatggttcga gagatacggg ccaatctatc aactgaagat acaaatcaga ttcaactaca 5640

acctccggag agcgttgaat cttcacaagt gctggataga gctgacgata actggatcga 5700

acaggatatt gactggaccc cgtttcttga aggtaagaaa tcttttccca tcttgaagtc 5760

acctcaaacc gaacgttagg aaattccaaa atgttttgat agtagtctac ttagtttcaa 5820

gttttgggtt tgtgtatact ttcactaata atatgcgtgg aaacattgca ggtcttgaaa 5880

aagagactag agatatactt ggataatcta ggtttaattt gtattaataa tgtaattaga 5940

ggtttaaatc atgtcctgta tgaagaattt acttttgtat caagtgtaat tcagaaccag 6000

agtgttgcaa tgaacttgta ctaa 6024

<210> 15
<211> 681
<212> DNA
<213> Bacteriophage lambda

<400> 15
atgacaccgg acattatcct gcagcgtacc gggatcgatg tgagagctgt cgaacagggg 60

gatgatgcgt ggcacaaatt acggctcggc gtcatcaccg cttcagaagt tcacaacgtg 120

atagcaaaac cccgctccgg aaagaagtgg cctgacatga aaatgtccta cttccacacc 180

ctgcttgctg aggtttgcac cggtgtggct ccggaagtta acgctaaagc actggcctgg 240

ggaaaacagt acgagaacga cgccagaacc ctgtttgaat tcacttccgg cgtgaatgtt 300

actgaatccc cgatcatcta tcgcgacgaa agtatgcgta ccgcctgctc tcccgatggt 360

ttatgcagtg acggcaacgg ccttgaactg aaatgcccgt ttacctcccg ggatttcatg 420

aagttccggc tcggtggttt cgaggccata aagtcagctt acatggccca ggtgcagtac 480

agcatgtggg tgacgcgaaa aaatgcctgg tactttgcca actatgaccc gcgtatgaag 540

cgtgaaggcc tgcattatgt cgtgattgag cgggatgaaa agtacatggc gagttttgac 600

gagatcgtgc cggagttcat cgaaaaaatg gacgaggcac tggctgaaat tggttttgta 660

tttggggagc aatggcgatg a 681

<210> 16
<211> 155
<212> DNA
<213> Artificial Sequence

<220>
<223> sgRNA comprising multiple stem-loops

<400> 16
gttttagagc taggccaaca tgaggatcac ccatgtctgc agggcctagc aagttaaaat 60

aaggctagtc cgttatcaac tttaaggagt ttatatggaa acccttaaag tggcaccgag 120

tcggtgctac cgccgacaac gcggtttttt ttttt 155

<210> 17
<211> 3237
<212> DNA
<213> Artificial Sequence

<220>
<223> Triple fusion of E.coli recA gene

<400> 17
atggacgaga acaagaagcg cgccctggcc gcggccctgg gacagatcga acgccaattc 60
ggcaaaggcg cggtcatgcg catgggcgac catgagcgcc aggcgatccc ggccatctcc 120
accggctccc tgggtctgga catcgccctc ggcatcggcg gcctgcccaa gggccggatc 180
gtcgagatct acggtccgga atcctcgggc aagaccaccc tgaccctctc ggtgatcgcc 240
gaggcccaga aacagggcgc cacctgtgcc ttcgtcgacg ccgagcacgc gctcgatccc 300
gactatgccg gcaagctggg cgtcaacgtc gacgacctgc tggtctccca gccggacacc 360
ggcgagcagg ccctggaaat caccgacatg ctggtgcgct ccaacgcggt cgacgtgatc 420
atcgtcgact ccgtggccgc gctggtaccc aaggccgaga tcgaaggcga gatgggcgac 480
gcccacgtcg gcctgcaggc acgcctgatg tcccaggcgc tgcgcaagat caccggcaat 540
atcaagaacg ccaactgcct ggtcatcttc atcaaccaga tccgcatgaa gatcggcgtc 600
atgttcggca acccggaaac caccaccggc ggtaacgcac tgaagttcta cgcctcggtc 660
cgcctggaca tccgtcgtac cggcgcggtg aaggaaggcg acgaggtggt gggtagcgaa 720
acccgcgtca aggtggtgaa gaacaaggtt tccccgccgt tccgccaggc cgagttccag 780
atcctctacg gtaagggcat ctaccgtacc ggcgagatca tcgatctggg cgtgcaattg 840
ggcctggtcg agaagtccgg cgcctggtac agctaccagg gcagcaagat cggccagggc 900
aaggcgaacg ccgccaagta cctggaagac aatccggaaa tcggttcggt actggagaag 960
accattcgcg accagttgct ggccaagagc ggcccggtga aggccgacgc cgaagaagtg 1020
gctgacgccg aagccgattc agagctcgga gaaggtcaag gacagggaca aggtccagga 1080
cgaggatacg catataagct tgacgagaac aagaagcgcg ccctggccgc ggccctggga 1140
cagatcgaac gccaattcgg caaaggcgcg gtcatgcgca tgggcgacca tgagcgccag 1200
gcgatcccgg ccatctccac cggctccctg ggtctggaca tcgccctcgg catcggcggc 1260
ctgcccaagg gccggatcgt cgagatctac ggtccggaat cctcgggcaa gaccaccctg 1320
accctctcgg tgatcgccga ggcccagaaa cagggcgcca cctgtgcctt cgtcgacgcc 1380
gagcacgcgc tcgatcccga ctatgccggc aagctgggcg tcaacgtcga cgacctgctg 1440
gtctcccagc cggacaccgg cgagcaggcc ctggaaatca ccgacatgct ggtgcgctcc 1500
aacgcggtcg acgtgatcat cgtcgactcc gtggccgcgc tggtacccaa ggccgagatc 1560
gaaggcgaga tgggcgacgc ccacgtcggc ctgcaggcac gcctgatgtc ccaggcgctg 1620
cgcaagatca ccggcaatat caagaacgcc aactgcctgg tcatcttcat caaccagatc 1680
cgcatgaaga tcggcgtcat gttcggcaac ccggaaacca ccaccggcgg taacgcactg 1740
aagttctacg cctcggtccg cctggacatc cgtcgtaccg gcgcggtgaa ggaaggcgac 1800
gaggtggtgg gtagcgaaac ccgcgtcaag gtggtgaaga acaaggtttc cccgccgttc 1860

cgccaggccg agttccagat cctctacggt aagggcatct accgtaccgg cgagatcatc 1920

gatctgggcg tgcaattggg cctggtcgag aagtccggcg cctggtacag ctaccagggc 1980

agcaagatcg gccagggcaa ggcgaacgcc gccaagtacc tggaagacaa tccggaaatc 2040

ggttcggtac tggagaagac cattcgcgac cagttgctgg ccaagagcgg cccggtgaag 2100

gccgacgccg aagaagtggc tgacgccgaa gccgattcag gatccggaga aggtcaagga 2160

cagggacaag gtccaggacg aggatacgca tatgcatgcg acgagaacaa gaagcgcgcc 2220

ctggccgcgg ccctgggaca gatcgaacgc caattcggca aaggcgcggt catgcgcatg 2280

ggcgaccatg agcgccaggc gatcccggcc atctccaccg gctccctggg tctggacatc 2340

gccctcggca tcggcggcct gcccaagggc cggatcgtcg agatctacgg tccggaatcc 2400

tcgggcaaga ccaccctgac cctctcggtg atcgccgagg cccagaaaca gggcgccacc 2460

tgtgccttcg tcgacgccga gcacgcgctc gatcccgact atgccggcaa gctgggcgtc 2520

aacgtcgacg acctgctggt ctcccagccg gacaccggcg agcaggccct ggaaatcacc 2580

gacatgctgg tgcgctccaa cgcggtcgac gtgatcatcg tcgactccgt ggccgcgctg 2640

gtacccaagg ccgagatcga aggcgagatg ggcgacgccc acgtcggcct gcaggcacgc 2700

ctgatgtccc aggcgctgcg caagatcacc ggcaatatca agaacgccaa ctgcctggtc 2760

atcttcatca accagatccg catgaagatc ggcgtcatgt tcggcaaccc ggaaaccacc 2820

accggcggta acgcactgaa gttctacgcc tcggtccgcc tggacatccg tcgtaccggc 2880

gcggtgaagg aaggcgacga ggtggtgggt agcgaaaccc gcgtcaaggt ggtgaagaac 2940

aaggtttccc cgccgttccg ccaggccgag ttccagatcc tctacggtaa gggcatctac 3000

cgtaccggcg agatcatcga tctgggcgtg caattgggcc tggtcgagaa gtccggcgcc 3060

tggtacagct accagggcag caagatcggc cagggcaagg cgaacgccgc caagtacctg 3120

gaagacaatc cggaaatcgg ttcggtactg gagaagacca ttcgcgacca gttgctggcc 3180

aagagcggcc cggtgaaggc cgacgccgaa gaagtggctg acgccgaagc cgattaa 3237

<210> 18
<211> 3207
<212> DNA
<213> Artificial Sequence

<220>
<223> Triple fusion of Arabidopsis rad51 gene

<400> 18
atgacgacga tggagcagcg tagaaaccag aatgctgtcc aacaacaaga cgatgaagaa 60

acccagcacg gacctttccc tgtcgaacag cttcaggcag caggtattgc ttctgttgat 120

gtaaagaagc ttagggatgc tggtctctgt actgttgaag gtgttgctta tactccgagg 180

aaggatctct tgcagattaa aggaattagt gatgccaagg ttgacaagat tgtagaagca 240

gcttcaaagc tagttcctct ggggttcaca agtgcgagcc agctccatgc tcagagacag 300

gaaattattc agattacctc tggatcacgg gagctcgata aagttctaga aggaggtatt 360

gaaactggtt ccatcacaga gttatatggt gagttccgct ctggaaagac tcagctgtgc 420

catacactgt gtgtgacttg tcaacttccc atggatcaag gaggtggaga gggaaaggcc 480

atgtacattg atgctgaggg aacattcagg ccacaaagac tcttacagat agctgacagg 540

tttggattaa atggagctga tgtactagaa aacgttgcct atgcgagggc gtataataca 600

gatcatcagt caaggctttt gcttgaagca gcatcaatga tgattgaaac aaggtttgct 660

ctcctgattg tcgatagtgc taccgctctc tacagaacag atttctctgg aaggggagag 720

ctttcggctc gacaaatgca tcttgcaaag ttcttgagaa gtcttcagaa gttagcagat 780

gagtttggtg tggctgttgt tataacaaac caagtagttg cgcaagtaga tggttcagct 840

ctttttgctg gtccccaatt taagccgatt ggtgggaata tcatggctca tgccaccaca 900

acaaggttgg cgttgaggaa aggaagagca gaggagagaa tctgtaaagt gataagctcg 960

ccatgtttgc cagaagcgga agctcgattt caaatatcta cagaaggtgt aacagattgc 1020

aaggatgagc tcggagaagg tcaaggacag ggacaaggtc caggacgagg atacgcatat 1080

aagcttatga cgacgatgga gcagcgtaga aaccagaatg ctgtccaaca acaagacgat 1140

gaagaaaccc agcacggacc tttccctgtc gaacagcttc aggcagcagg tattgcttct 1200

gttgatgtaa agaagcttag ggatgctggt ctctgtactg ttgaaggtgt tgcttatact 1260

ccgaggaagg atctcttgca gattaaagga attagtgatg ccaaggttga caagattgta 1320

gaagcagctt caaagctagt tcctctgggg ttcacaagtg cgagccagct ccatgctcag 1380

agacaggaaa ttattcagat tacctctgga tcacgggagc tcgataaagt tctagaagga 1440

ggtattgaaa ctggttccat cacagagtta tatggtgagt tccgctctgg aaagactcag 1500

ctgtgccata cactgtgtgt gacttgtcaa cttcccatgg atcaaggagg tggagaggga 1560

aaggccatgt acattgatgc tgagggaaca ttcaggccac aaagactctt acagatagct 1620

gacaggtttg gattaaatgg agctgatgta ctagaaaacg ttgcctatgc gagggcgtat 1680

aatacagatc atcagtcaag gcttttgctt gaagcagcat caatgatgat tgaaacaagg 1740

tttgctctcc tgattgtcga tagtgctacc gctctctaca gaacagattt ctctggaagg 1800

ggagagcttt cggctcgaca aatgcatctt gcaaagttct tgagaagtct tcagaagtta 1860

gcagatgagt ttggtgtggc tgttgttata acaaaccaag tagttgcgca agtagatggt 1920

tcagctcttt ttgctggtcc ccaatttaag ccgattggtg ggaatatcat ggctcatgcc 1980

accacaacaa ggttggcgtt gaggaaagga agagcagagg agagaatctg taaagtgata 2040

agctcgccat gtttgccaga agcggaagct cgatttcaaa tatctacaga aggtgtaaca 2100

gattgcaagg atggatccgg agaaggtcaa ggacagggac aaggtccagg acgaggatac 2160

gcatatgcat gcatgacgac gatggagcag cgtagaaacc agaatgctgt ccaacaacaa 2220

gacgatgaag aaacccagca cggacctttc cctgtcgaac agcttcaggc agcaggtatt 2280

gcttctgttg atgtaaagaa gcttagggat gctggtctct gtactgttga aggtgttgct 2340

tatactccga ggaaggatct cttgcagatt aaaggaatta gtgatgccaa ggttgacaag 2400

attgtagaag cagcttcaaa gctagttcct ctggggttca caagtgcgag ccagctccat 2460

gctcagagac aggaaattat tcagattacc tctggatcac gggagctcga taaagttcta 2520

gaaggaggta ttgaaactgg ttccatcaca gagttatatg gtgagttccg ctctggaaag 2580

actcagctgt gccatacact gtgtgtgact tgtcaacttc ccatggatca aggaggtgga 2640

gagggaaagg ccatgtacat tgatgctgag ggaacattca ggccacaaag actcttacag 2700

atagctgaca ggtttggatt aaatggagct gatgtactag aaaacgttgc ctatgcgagg 2760

gcgtataata cagatcatca gtcaaggctt ttgcttgaag cagcatcaat gatgattgaa 2820

acaaggtttg ctctcctgat tgtcgatagt gctaccgctc tctacagaac agatttctct 2880

ggaaggggag agctttcggc tcgacaaatg catcttgcaa agttcttgag aagtcttcag 2940

aagttagcag atgagtttgg tgtggctgtt gttataacaa accaagtagt tgcgcaagta 3000

gatggttcag ctctttttgc tggtccccaa tttaagccga ttggtgggaa tatcatggct 3060

catgccacca caacaaggtt ggcgttgagg aaaggaagag cagaggagag aatctgtaaa 3120

gtgataagct cgccatgttt gccagaagcg gaagctcgat ttcaaatatc tacagaaggt 3180

gtaacagatt gcaaggatta actagtg 3207

<210> 19
<211> 34
<212> DNA
<213> Artificial Sequence

<220>
<223> MS2-derived stem-loop for binding

<400> 19
ggccaacatg aggatcaccc atgtctgcag ggcc 34

<210> 20
<211> 25
<212> DNA
<213> Artificial Sequence

<220>
<223> PP7-derived stem-loop for binding

<400> 20
taaggagttt atatggaaac cctta 25

<210>  21
<211>  17
<212>  DNA
<213>  Artificial Sequence

<220>
<223>  P22-derived stem-loop for binding B-box

<400>  21
accgccgaca acgcggt                                                    17


<210>  22
<211>  385
<212>  DNA
<213>  Bacteriophage MS2

<400>  22
atggcttcaa actttactca gttcgtgctc gtggacaatg gtgggacagg ggatgtgaca      60

gtggctcctt ctaatttcgc taatggggtg gcagagtgga tcagctccaa ctcacggagc     120

caggcctaca aggtgacatg cagcgtcagg cagtctagtg cccagaagag aaagtatacc     180

atcaaggtgg aggtccccaa agtggctacc cagacagtgg gcggagtcga actgcctgtc     240

gccgcttgga gatcctacct gaacatggaa ctcactatcc caattttcgc taccaattct     300

gactgtgaac tcatcgtgaa ggcaatgcag gggctcctca aagacggcaa tcctatccct     360

tccgccatcg ccgctaactc aggca                                           385


<210>  23
<211>  387
<212>  DNA
<213>  Bacteriophage PP7

<400>  23
atgtccaaaa ccatcgttct ttcggtcggc gaggctactc gcactctgac tgagatccag      60

tccaccgcag accgtcagat cttcgaagag aaggtcgggc ctctggtggg tcggctgcgc     120

ctcacggctt cgctccgtca aaacggagcc aagaccgcgt atcgagtcaa cctaaaactg     180

gatcaggcgg acgtcgttga ttgctccacc agcgtctgcg gcgagcttcc gaaagtgcgc     240

tacactcagg tatggtcgca cgacgtgaca atcgttgcga atagcaccga ggcctcgcgc     300

aaatcgttgt acgatttgac caagtccctc gtcgcgacct cgcaggtcga agatcttgtc     360

gtcaaccttg tgccgctggg ccgttaa                                         387


<210>  24
<211>  303
<212>  DNA
<213>  Bacteriophage P22

<400>  24
atgacggtta tcacctacgg gaagtcaacg tttgcaggca atgctaaaac tcgccgtcat      60

gagcggcgca gaaagctagc catagagcgc gacaccatct gcaatatcat cgattcaatt 120

tttggctgcg atgctcctga tgcttctcag gaagttaaag ccaaaagaat tgaccgtgtc 180

accaaagcca tttcgcttgc cggaacgcgt cagaaggaag ttgaaggagg atctgtactt 240

cttccaggcg tagcacttta cgcggctggt catcgtaaga gcaaacaaat aacagcgagg 300

taa 303

<210> 25
<211> 2753
<212> DNA
<213> Artificial Sequence

<220>
<223> MSH2 dominant-negative allele gene sequence

<400> 25
atggctgggt taaggcagga tcttagacag catctgaagc gaatctcaga tgttgagagg 60

cttttgcgca gtctcgagag aagaagaggt gggttacagc acattattaa actctatcag 120

gtactttccg cacttcaatc tgcttctctc aatgttaaca aaattgcatt ttcattgtcc 180

taaatgtgtt tatgcaactc tgaagttata ggtatgttat taagttcatt actaattaag 240

tcttcatctt ttctctgcag tcagctataa ggcttccctt catcaaaaca gctatgcaac 300

agtacaccgg agaattcgca tcactcatca gcgagaggta cctgaaaaag cttgaggctt 360

tatcagatca agatcacctt ggaaagttca tcgatttggt tgagtgctct gtagatcttg 420

accagctaga aaatggagaa tacatgatat cttcaaacta cgacaccaaa ttggcatctc 480

tgaaagatca gaaagaattg ctggagcagc agattcacga attgcacaaa aagacagcga 540

tagaacttga tcttcaggtc gacaaggctc ttaaacttga caaagcagcg caatttgggc 600

atgtcttcag gatcacgaag aaggaagagc caaagatcag gaagaagctg acgacacagt 660

ttatagtgct ggagactcgc aaagacggag tgaagttcac aaacacaaag ctaaaaaaac 720

tgggcgacca gtaccaaagt gttgtggatg attataggag ctgtcaaaag gagctcgttg 780

atcgtgtagt tgagactgtt accagcttct ctgaggtatg tttagttatt catattaagc 840

attggactgt tacagaattg gttgtttaaa atcatagtaa actatatgtg gaatttatat 900

gtatattgta tggttatagg tatttgagga cttagctggg ttactttctg aaatggatgt 960

tttgttaagc tttgctgatt tggctgccag ttgccctact ccatactgta ggccagaaat 1020

cacctctttg gttagtacaa tctcaagttg attattttgt tctgaaaatg aatagttttt 1080

tctttccaag tttatgacat aatgttgaga gcacggttaa taaattgtag gatgctggag 1140

atattgtact agaaggaagc agacatccat gtgtagaagc tcaagattgg gtgaatttca 1200

taccaaatga ttgcagactc gtaagtattg aatgtggtaa ataaactgag acgtctttgt 1260

ttttcttgtt tcccttttga cttgaacaaa tacttgtttg ccctttactg ttctttgaaa 1320

tcagatgaga gggaagagtt ggtttcaaat agtaacaggg cctaacatgg gagataagtc 1380

cactttcatc cgccaggtat gatgatttcc tctagttcag ttttgcttca tagacgtatg 1440

actaaagtcg gtttccggcc attataaatc ccaggttggt gtgattgtgc tgatggctca 1500

agttggttcc tttgttcctt gtgataaagc atcaatttcc ataagagact gcatctttgc 1560

ccgtgtagga gcaggcgatt gccaagtgag tttaagttta gccctcaatg aacgaaaaac 1620

tgctgatatc ctgaacaccc ttattccaac tttttttcct ttggtgtgtt agctgcgtgg 1680

agtgtcaact tttatgcaag aaatgcttga aaccgcatcg atattgaaag gcgctactga 1740

taagtcactg ataattatcg atgaacttgg tcgtggaaca tcaacttatg atggttttgg 1800

ttagtttctc tgcaatttct cttctttcat ttggatgttt ttagtaagtt ttctattata 1860

tattcatttt tatggtcata tgtgagattt cagtgctctt gacatcatcg tggtgaatat 1920

atcaggttta gcttgggcta tatgtgagca tctggttcaa gtgaaaagag caccaactct 1980

gtttgctact cacttccatg aacttactgc cttggctcaa gcaaactctg aggtctctgg 2040

taacactgtt ggtgtggcaa acttccatgt cagcgctcac attgacactg aaagccgcaa 2100

actcaccatg ctttacaagg tctggtttat aaattaaaaa attgctgatc tgttgcagtt 2160

aaaagtgtct ctgtttttat gtttaatcta aattacttat ttgattttct tacaaagatg 2220

aaattgaaat taattttgtg tggtgtgttg tttgtctggt taggttgaac caggggcctg 2280

tgaccagagc tttgggattc atgtggcgga atttgccaac ttccctgaaa gcgtcgtggc 2340

cctcgcaaga gagaaagctg cagagctgga agatttctct ccctcctcga tgataatcaa 2400

caatgaggtc ttgattcatt tccccctttg tttttggttg atgatggaat cattctatca 2460

ttcacccatt ctgcagttta tgctatatta ttataaatct atgtgacaaa gatttaattc 2520

tcgtattgtt gtttgcagga gagtgggaag agaaagagca gagaagatga tccagatgaa 2580

gtatcaagag ggcagagcga agctcacaag tttctgaaag agtttgcagc gatgccactt 2640

gataaaatgg agcttaaaga ttcacttcaa cgggtacgtg agatgaaaga tgagctagag 2700

aaagatgctg cagactgcca ctggctcagg cagtttctgt gaagaacccc tga 2753

## Description

The present invention relates to methods, reagents and compositions for providing more accurate and reliable genetic modification. The invention further provides methods, reagents and compositions for in vivo genetic modification of the genome of a non-animal cell or organism. Furthermore, the present invention relates to uses of the said methods, reagents and compositions for introducing desirable traits to non-animal organisms or ameliorating or removing non-desirable traits in these organisms including in the treatment of diseases and production of transgenic organisms.

In recent times genetic modification by way of random mutagenesis has given way to directed mutagenesis of particular nucleotide sequences using sequence-specific protein complexes.

Examples of such protein complexes include zinc-finger nucleases (ZFN), transcription activator-like effector nucleases (TALENs), complexes derived from the CRISPR-Cas9 system of *Streptococcus pyrogenes* and other bacteria, and CRISPR-Cpf1.

ZFNs and TALENs are both protein nucleases whose protein structure allows them to interact with and recognise a particular DNA sequence before cutting the DNA at a defined location. Thus cutting a particular DNA sequence requires a uniquely designed ZFN or TALEN protein.

In contrast, the CRISPR-Cas9 and CRISPR-Cpf1 systems use a single protein whose activity is directed by an RNA cofactor whose nucleotide sequence defines the location that the endonuclease will act at to produce a double strand break.

Thus, all of these protein complexes act by making a DNA double strand break at a predefined DNA sequence. This double strand break is then normally repaired by the non-homologous end joining (NHEJ) pathway.

Repair by NHEJ is highly efficient and rapid but is more error-prone than the alternative pathway for repair of DNA double-stranded breaks which is homology-directed repair (HDR). Consequently, a proportion of NHEJ pair of events will cause insertion or deletion of nucleotides at the break site. Such insertion or deletion events are known as 'indels'.

Alternatively, larger genetic modifications are enabled by the presence of a donor DNA molecule in the vicinity of an artificially-created DNA double-stranded break. In this instance HDR of the induced DSB causes repair of the DSB with using the sequence of the donor molecule. In this way specific modifications can be made and short sequence insertions are also possible. One example of such a donor and a vector for producing large amounts of such donor molecules is disclosed in WO/2010/084331.

However, the efficiency of genetic modification using this approach is low because most repair of double strand breaks proceeds via the more rapid NHEJ pathway.

Furthermore, while the above-mention protein complexes are directed to specific sequences their endonuclease activity has been known to act at other sites. Such "off-site breaks" are particularly a problem as NHEJ is more error prone.

Thus, there exists a need for alternative and preferably improved methods and reagents for sequence-specific modification of nucleic acid sequences and of DNA sequences in particular. Furthermore, there is a need for techniques and reagents that more reliably and efficiently yield the desired genetic modification. Additionally, there is a need for techniques and reagents that reliably allow insertion of longer DNA sequences at a predefined locus.

An object of the present invention is to provide reagents and techniques for using these reagents that allow more reliable, efficient and accurate modification and/or mutation of a target genome at specific loci within the genome.

There are provided herein proteins and protein-nucleic acid complexes that provide improved transformation efficiencies and methods for carrying out such transformations. Furthermore the said methods, reagents and compositions may be used for introducing desirable traits to plants, algae, bacteria and other non-animal organisms or ameliorating or removing non-desirable traits in these organisms including in the treatment of diseases.

Accordingly, the present invention provides a nucleic acid encoding a first fusion protein comprising an endonuclease domain and a binding domain for an origin of replication.

Functionally significant domains or regions of different proteins or polypeptides may be combined for expression from an encoding nucleic acid as a fusion protein. For example, particularly advantageous or desirable properties of different proteins or polypeptides may be combined in a hybrid protein, such that the resultant expression product, may include fragments of various parent proteins or polypeptides.

In the fusion proteins described herein the domains of the fusion proteins are preferably joined together via linker peptides. The particular choice of linker will depend on the constituent domains of the fusion protein. The suitability and choice of appropriate linker peptides is discussed in Chen et al. (Adv Drug Deliv Rev. 2013; 65(10): 1357-1369).

The endonuclease may cleave a target nucleic acid molecule in a sequence specific manner. The sequence specific cleavage of the nucleic acid molecule may be double or single stranded (including 'nicking' of duplexed nucleic acid molecules. Double stranded cleavage may yield blunt ends or overhanging termini (5'or 3' overhangs). The sequence specific nuclease preferably act as a monomer but may act as a dimer or multimer. For instance a homodimer wherein both monomers make single strand nicks at a target site can yield a double-strand break in the target molecule. Preferably the cleavage event makes a double-stranded break in the target molecule.

Examples of sequence-specific endonucleases include zinc-finger nucleases (ZFN), transcription activator-like effector nucleases (TALENs), complexes derived from the CRISPR-Cas9 system of *Streptococcus pyrogenes* and other bacteria, and CRISPR-Cpf1.

A nucleic acid molecule may comprise double- or single-stranded DNA or RNA. The nucleic acid molecule may also comprise a DNA-RNA duplex. Preferably the nucleic acid molecule is double-stranded DNA. Preferably the cleavage event makes a double-stranded DNA break in the target molecule.

Preferably the endonuclease is a DNA endonuclease and most preferably this is Cas9. This may be Cas9 from *Streptococcus pyrogenes* or a homologous or functionally equivalent enzyme from another bacteria.

The fusion protein may comprise an endonuclease and a component of the replication initiation complex or replication complex.

The components of the replication initiation complex or replication complex are necessarily associated with origins of replication and may be covalently attached thereto or to the elongating nucleic acid molecule. Suitably the origin of replication is derived from a virus. Most suitably the virus is geminivirus or another member of the geminiviridae.

Geminivirus *Rep* protein (GV-Rep) binds to the geminivirus origin of replication and thus becomes covalently linked to the ssDNA strand of donor DNA produced by rolling circle replication initiated at the origin of replication. Thus the newly replicated donor DNA molecule is covalently linked to the first fusion protein and is necessarily brought into close proximity to the site of the double-stranded DNA break caused by the endonuclease.

The invention further provides a nucleic acid encoding a second fusion protein comprising a 5' to 3' DNA exonuclease domain and an RNA binding domain.

Both Zalatan et al. (Cell (2015) 160, 339-350) and the CRISPRainbow system described initially by Ma et al. (Nat Biotechnol. 2016 APR 18. doi: 10.1038/nbt.3526) utilise a modified sgRNA containing 3' RNA hairpin aptamers that bind uniquely labelled RNA binding proteins (SEQ ID NO: 16). Thus the sgRNA is functionalised so that it can be used to locate fusion proteins comprising binding domains for the aptamers in association with the sgRNA and hence the endonuclease it is associated with.

The action of the second fusion protein may be for inhibition of NHEJ during transformation of a cellular genome so as to promote HDR. The effect of such 5' to 3' resection on DNA double-strand breaks is to suppress religation of DNA breaks (i.e. by blocking NHEJ), by producing a substrate that is less suitable for NHEJ but is significantly more suitable for HDR. The action of the second fusion protein may be for inhibition of NHEJ during transformation of a genome so as to promote HDR.

The exonuclease may be a dsDNA exonuclease. The exonuclease may be Exo1. Exonuclease I (exol) is a 5' to 3' exonuclease and is involved in recombination, double-strand break repair, the MMS2 error-free branch of the post replication repair (PRR) pathway and DNA mismatch repair. Preferably the exo1 is the exo1 protein from bacteriophage λ (SEQ ID NO: 15). Without wishing to be bound by theory, this enzyme can produce approximately 100-150 bp 3' overhangs at dsDNA break sites during methods of the invention.

The invention further provides nucleic acid encoding a third fusion protein comprising a recombination inducing domain and an RNA binding domain.

The recombination domain may be a protein or polypeptide that interacts with a target or donor nucleic acid molecule in order to catalyse modification of the nucleotide sequence of the target nucleic acid with reference to the nucleotide sequence of the donor nucleic acid molecule.

Modification of the target nucleic acid may be by way of insertion of all or a part of the sequence of the donor nucleic acid molecule or substitution of all or a part of the sequence of the donor nucleic acid molecule for a homologous section of the target nucleic acid molecule. In this way deletions, insertions, frameshift mutations and single nucleotide mutations may be achieved.

The recombination inducing event caused or mediated by the recombination inducing domain may be initiating or catalysing strand exchange between the target and donor nucleic acid molecules.

The recombination domain may be RecA from *E. coli* or a homologue thereof, Rad51 or a homologue thereof from a plant or another organism, or an annealase from such as bacteriophage λ recombination protein *beta* (BET; Redβ) or a homologue thereof. Studies of phage lambda *in vivo* have indicated that bacteriophage λ beta protein can catalyse steps that are central to both the strand annealing and strand invasion pathways of recombination. A homologous protein in this case may have functional or sequence homology, preferably functional homology.

Preferably the recombination domain is a trimer of RecA (SEQ ID NO: 17) or Rad51 monomers (SEQ ID NO: 18). Most preferably the monomers are joined by peptide linkers. Use of a trimer of monomers for the recombination domain is advantageous because this allows binding of a turn of the nucleic acid helix in order to more efficiently initiate strand exchange and hence HDR

The invention further provides a nucleic acid encoding a fourth fusion protein comprising a domain comprising an inhibitor of the mismatch repair pathway; and an RNA binding domain.

MSH2 and MSH6 are proteins involved in base mismatch repair and the repair of short insertion/deletion loops. The MSH2 dominant-negative mutant (SEQ ID NO: 25) competes with MSH2 binding to mismatches thus blocking the ability of the wild-type MSH2 protein to repair these mismatches. A dominant negative allele of MSH6 is also known and may be used in the same way as the dominant negative allele of MSH2.

The second, third and fourth fusion proteins may bind to the RNA component of an RNA-guided endonuclease for use in transformation mediated by the RNA-guided endonuclease. Preferably an RNA component is a tracrRNA molecule or domain for use in transformation using the CRISPR-Cas9 system.

The invention also provides a method of transforming the genome of a non-animal cell comprising the steps of:
a. expressing an RNA-guided endonuclease in the cell;
b. expressing in the cell or introducing into the cell a sequence specific guide RNA to direct cleavage by the endonuclease domain to a specific locus
c. expressing in the cell the nucleic acid encoding the second fusion protein or introducing the second fusion protein into the cell.

Thus the invention provides a system with multiple features that may be used separately or in concert. These features include:
a. Induction of dsDNA break using the sequence-specific endonuclease of the first fusion protein.
b. Delivery of donor nucleic acid molecule to within close proximity of the induced DNA break by associating the donor nucleic acid molecule with the origin-binding domain of the first fusion protein.
c. Suppression of religation of DNA breaks (i.e. blocking NHEJ), preferably, as noted above, by 5' to 3' resection of double-stranded DNA breaks in order to produce a substrate that is not suitable for NHEJ but is more suitable for HDR;
d. Delivery of recombinase to the induced dsDNA break.
e. Suppression of the mismatch repair pathway in the vicinity of the induced dsDNA breaks by providing an inhibitor of this pathway. As noted above this is preferably a fusion protein comprising a dominant negative suppressor protein of the mismatch repair system.
Features c, d, and e are supplied to the HDR complex by their being provided in the form of the second, third and fourth fusion proteins, i.e. each comprises a domain that binds to an aptamer engineered to be part of the sgRNA that guides the endonuclease activity of the first fusion protein (e.g. the sgRNA of SEQ ID NO: 16).

The second, third and fourth fusion proteins each comprises a domain that binds to an aptamer engineered to be part of the sgRNA that guides the endonuclease activity of an RNA-guided endonuclease. Therefore the second, third and fourth fusion proteins may be used in concert with an RNA-guided endonuclease other than the first fusion protein, such as Cas9 or Cpf1.

One advantage flowing from use of any or all of the first, second, third and/or fourth fusion proteins of the invention is more reliable and efficient genetic modification.

A further advantage is that use of any or all of the first, second, third and/or fourth fusion proteins of the invention allows for insertion of longer DNA sequences at a locus or loci acted on by a sequence-guided endonuclease than has previously been reported.

The invention also provides a method of modifying the genome of a non-animal organism or cell comprising:
a. expressing in the cell the nucleic acid encoding the second fusion protein or introducing the second fusion protein into the cell; and
b. expressing in the cell or introducing into the cell a donor nucleic acid molecule comprising an origin of replication.

Non-animal organisms in the context of the present disclosure may be prokaryotes (bacteria and archaea), algae, plants or any other non-animal organism including protists and fungi. Preferably, the non-animal organisms are plants along with any part or propagule thereof, seed, selfed or hybrid progeny and descendants.. The plants may be monocot or dicot plants. Suitably the plants are *Arabidopsis,* tobacco, rice or a transgenic crop plant. Examples of suitable transgenic crop plants include tobacco (*Nicotiana tabacum*) and other Nicotiana species, carrot, vegetable and oilseed Brassicas, melons, Capsicums, grape vines, lettuce, strawberry, sugar beet, wheat, barley, (corn) maize, rice, soya bean, peas, sorghum, sunflower, tomato, cotton, and potato. The non-animal organisms may be algae.

The donor nucleic acid molecule may comprise:
a. a donor nucleic acid sequence;
b. flanking nucleic acid sequences located 5' and 3' to the donor nucleic acid sequence;
c. an origin of replication 5' to the 5' flanking nucleotide sequence, and
d. a replication terminator 3' to the 3' flanking nucleotide sequence.

The flanking nucleic acid sequences located 5' and 3' to the donor nucleic acid sequence may be homologous to loci flanking the target loci, sequence or nucleotide.

The replication terminator may be a non-functioning origin of replication that is still capable of terminating replication when a replication fork reaches it. In a specific example, a geminivirus origin of replication is nicked by the Rep protein at a particular location on a stem loop characteristic of the origin of replication. As long as the stem loop is present and correctly nicked then replication will be terminated at that location. Other sequence elements of the origin are not essential for termination and therefore can be omitted from the replication terminator in this example.

However, the nick at the replication terminator derived from such an origin of replication (in, for instance geminiviruses) may still be competent for relegation of the nicked stem loops at the active origin of replication and the downstream terminator/origin of replication. In this way a nucleic acid circle with an active origin of replication is provided and may be actively replicated by rolling circle replication or another mode of replication.

Rolling circle replication of the donor DNA acid molecule has the advantage of providing a large amount of donor DNA nucleic acid. Provision of a relatively large amount of donor nucleic acid molecule means that the probability of the successful transformation is raised.

The methods described herein may comprise introducing a double strand break into the genome in the presence of an exogenous donor nucleic acid molecule comprising a donor nucleic acid sequence as a template for modifying the genome or as an exogenous sequence to be integrated into the genome and a DNA repair mechanism modifies the genome via homology-directed repair (HDR).

The method may further comprise the step or effect of suppressing non-homologous end joining (NHEJ) repair of a DNA double-strand break to promote repair of the break by HDR by expressing in the cell a nucleic acid encoding the second fusion protein or introducing the second fusion protein into the cell.

The methods described herein may comprise introducing a double strand break into the genome in the presence of an exogenous donor nucleic acid molecule comprising a donor nucleic acid sequence as a template for modifying the genome or as an exogenous sequence to be integrated into the genome and a DNA repair mechanism modifies the genome via homology-directed repair (HDR) the method comprising:
suppressing non-homologous end joining (NHEJ) repair of the break to promote repair by HDR by expressing in the cell a nucleic acid encoding the second fusion protein or introducing the second fusion protein into the cell.

The method may further comprise the steps of:
a. expressing in the cell or introducing into the cell a sequence specific guide RNA to direct cleavage by the endonuclease domain to a specific locus; and
b. expressing in the cell one or more nucleic acids of the second, third and fourth fusion proteins or introducing one or more of the second, third and fourth fusion proteins into the cell.

The method may further comprise the steps of:
a. expressing in the cell one or more nucleic acids encoding the second, third and fourth fusion proteins; or
b. introducing into the cell one or more of the second, third and fourth fusion proteins.

The method may further comprise the steps of expressing in the cell two or more nucleic acids encoding the second, third and fourth fusion proteins or introducing into the cell two or more of the second, third and fourth fusion proteins, wherein the RNA binding protein domains of the respective fusion proteins bind to different RNA sequences.

In this way the first fusion protein may be using in concert with the second, third and fourth fusion proteins for transformation of a non-animal cell or organism in concert with an RNA-guided endonuclease.

Expression of the first, second, third and fourth fusion proteins during a method of modifying the genome as describe herein may be via inducible and/or transient expression.

Various methods for introducing nucleic acids encoding the fusion proteins and nucleic acids of the invention are envisaged these include electroporation and infiltration in order to introduce proteins, DNA and/or RNA.

The invention also provides a first fusion protein comprising an endonuclease and a component of the replication initiation complex or replication complex.

The invention further provides a second fusion protein comprising a 5' to 3' DNA exonuclease domain and an RNA binding domain.

The invention also provides a third fusion protein comprising a recombination inducing domain and an RNA binding domain

The invention further provides a fourth fusion protein comprising a domain comprising an inhibitor of the mismatch repair pathway and an RNA binding domain.

The invention further provides for use of the first fusion protein, or a nucleic acid encoding the first fusion protein in transformation of a non-animal organism or cell.

The invention further provides for use of the second fusion protein, or a nucleic acid encoding the second fusion protein in transformation of a non-animal organism or cell using an RNA-guided endonuclease.

The invention also provides for use of the first fusion protein, or a nucleic acid encoding the first fusion protein in concert with the second, third and fourth fusion proteins in transforming a non-animal organism or cell using an RNA-guided endonuclease.

The invention further provides vectors comprising the nucleic acids of the invention. Such vectors may be suitable for modification in vitro or in vivo.

Vectors of the invention capable of expressing products encoded on nucleotides of the invention may also be suitable for expression in a host cell or cell-free system. Suitably the host cell may be a cultured plant cell, yeast cell or bacterial cell, e.g. *Escherichia coli.* Compositions and products of the invention may be obtained by methods comprising expressing such encoded products in a suitable host cell or cell-free system.

The invention also provides the methods, reagents and compositions disclosed herein for use in the treatment of disease in non-animal organisms.

The invention also provides uses of the methods, reagents and compositions disclosed herein for introducing desirable genetic characteristics to non-animal organisms or ameliorating or removing non-desirable genetic characteristics in these organisms.

The invention also provides uses of the methods, reagents and compositions disclosed herein for introducing desirable heritable characteristics to non-animal organisms or ameliorating or removing non-desirable inherited characteristics in these organisms.

Accordingly, the invention also provides non-animal transgenic organisms, transgenic cells thereof and transgenic non- animal cell lines. Organisms which include a transgenic cell according to the invention are also provided.

The invention further provides methods of treating disease or other conditions of non-animal organisms or cells by utilising the methods, reagents and compositions disclosed herein.

The invention is now illustrated in specific embodiments with reference to the accompanying drawings in which:

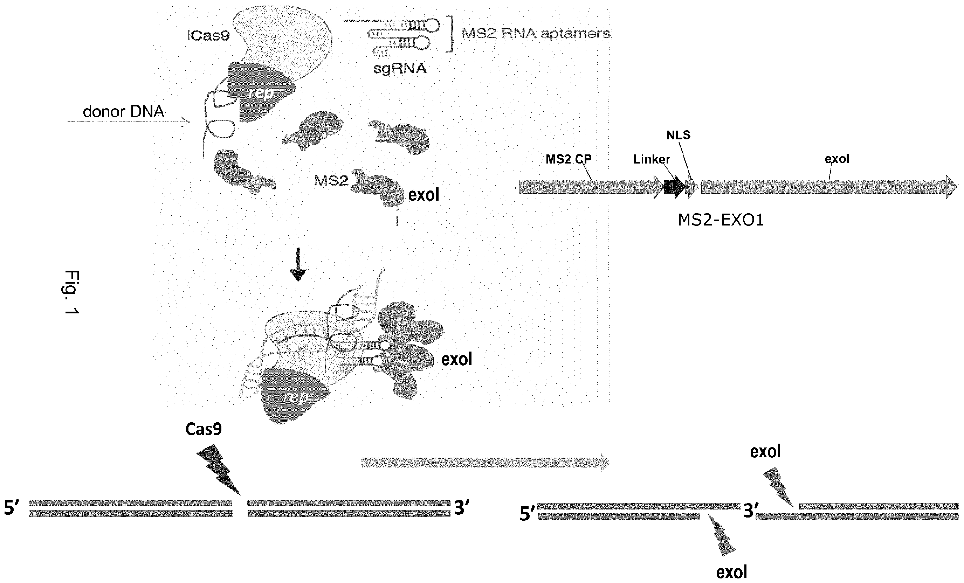

- Fig. 1: shows a schematic representation of inducing a DNA double strand break with Cas9 protein (Cas9) and resecting the DNA DSB with exo1. The exo1-MS2 fusion protein is engineered to bind to the single guide RNA (sgRNA) via aptamer loops on the sgRNA that bind to the MS2 domain (part of SEQ ID NO: 22).

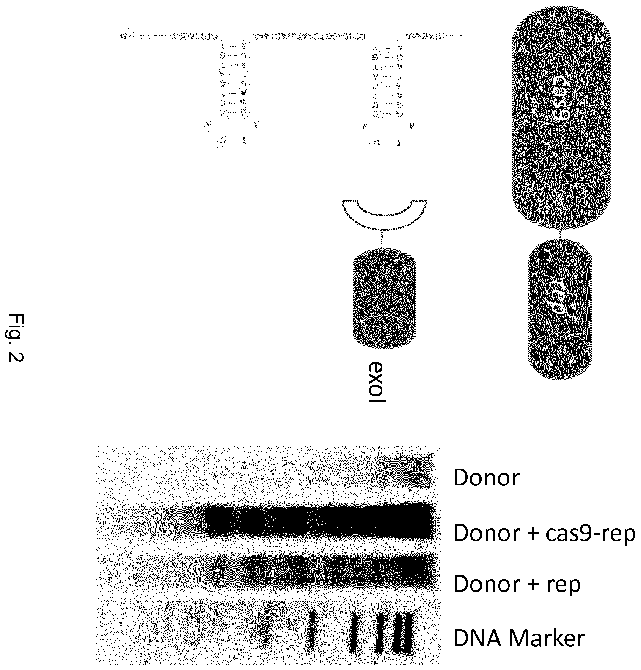

- Fig. 2: shows a schematic representation of a cas9-Rep (virus replication associated protein) fusion protein (SEQ ID NO: 14) and an exo1-MS2 fusion protein showing its binding to an aptamer loop (SEQ ID NO: 19). Also shown is an electrophoresis gel demonstrating the activity of the cas9-Rep fusion protein.

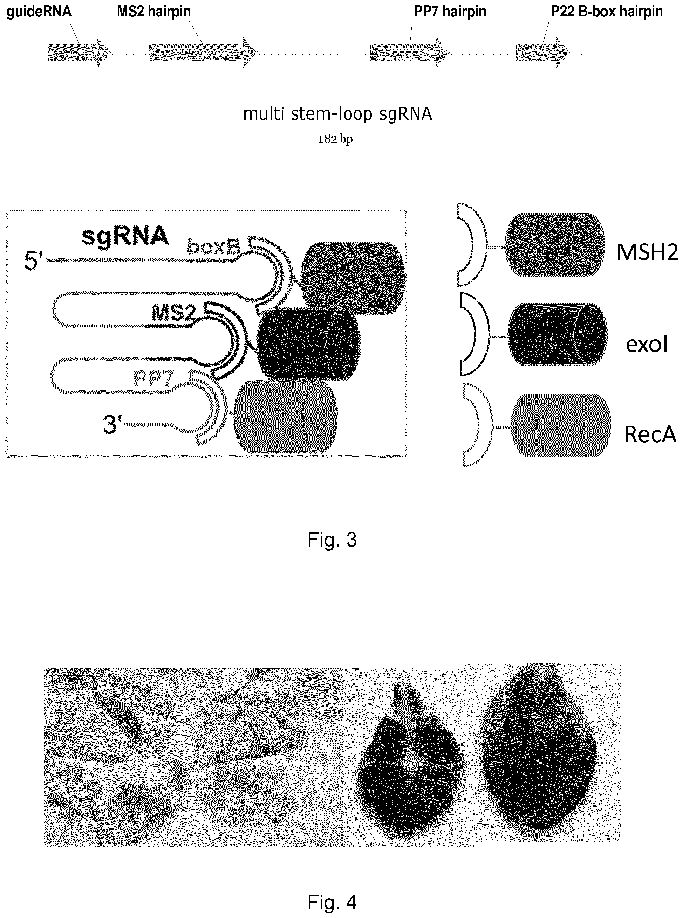

- Fig. 3: shows the design of a multi stem-loop sgRNA with hairpins from different bacteriophages (MS2, PP7 and P22 bacteriophages; SEQ ID NO: 19-21).
- Fig. 4: shows examples of tobacco leaves in which a *uidA* with a two-base pair frame shift mutation (SEQ ID NO: 2) is repaired. The transgenic lines were assayed for GUS activities as described by McCabe et al., (Nature Biotechnology, 1988, 6, 923-926). Blue colour indicates repair of the mutated *uidA* gene; the extent of the blue colour indicates the extent of the repair.

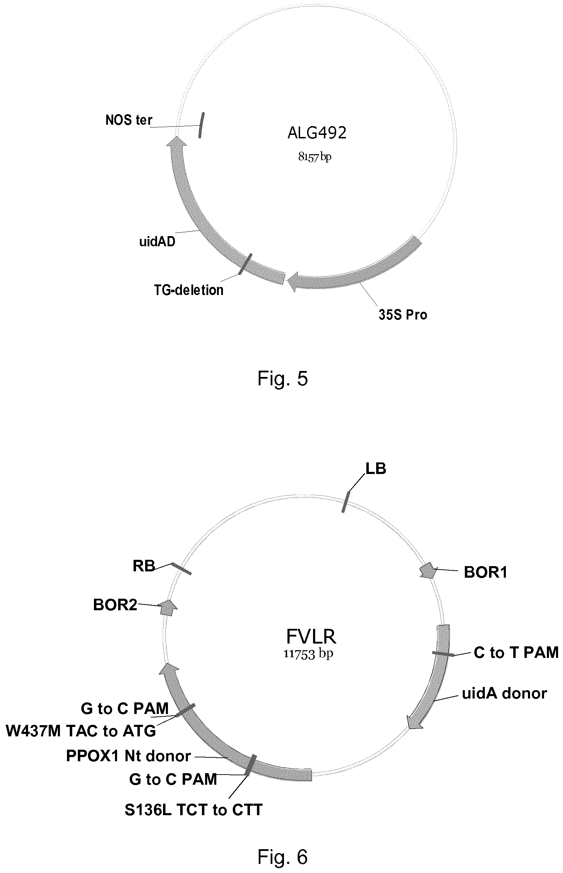

- Fig. 5: shows a vector containing mutated *uidA* gene (SEQ ID NO: 2) from *E. coli* under the 35S promoter (SEQ ID NO: 1) used for stable transformation of tobacco and subsequent utilisation of stably transformed tobacco lines for gene editing.
- Fig. 6: shows a vector for delivery of donor molecules for repair of *uidA* (SEQ ID NO: 4) in tobacco ALG492 stable transgenic lines, and mutagenesis of PPOX1 gene from tobacco to induce herbicide resistance. BOR1 and BOR2 denote viral origins of replication from beet top curly virus (BCTV) (SEQ ID NO: 7). 'uidA donor' contains sequence for repair of a 2 bp deletion with modification in a PAM triplet. 'PPOX1 Nt donor' denotes a sequence for generation of mutations in endogenous tobacco PPOX1 gene (SEQ ID NO: 5) to induce herbicide resistance to oxyfluorfen or butafenacil herbicides. Two mutations were designed to introduce herbicide resistance: S136L and W437M. PAM triplets were modified to prevent the donor from being cut by cas9.

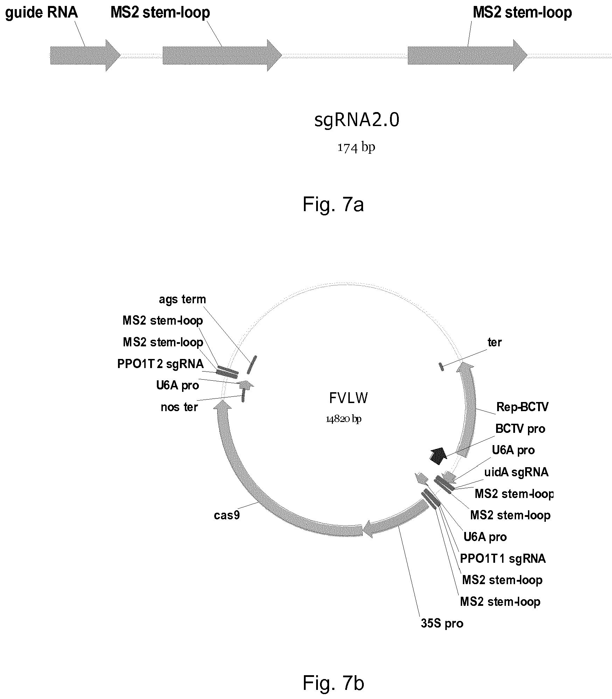

- Fig. 7: (a) shows the design of sgRNA for precise targeting of proteins to the double-stranded DNA break. Two stem-loops from bacteriophage MS2 (SEQ ID NO: 19) were introduced into the sgRNA. (b) shows the FVLW vector containing cas9 (SEQ ID NO: 8) and Rep-BCTV (SEQ ID NO: 7) cassettes for expression in plants. The construct also has sgRNAs with guide for the uidA gene (SEQ ID NO: 10) and two additional sgRNAs with guides for targeting the tobacco PPOX1 gene (SEQ ID NOs: 10 and 11). The *Arabidopsis* U6A promoter (SEQ ID NO: 9) was used for expression of sgRNAs containing a gene-specific guide and sgRNAs with two MS2 loops (SEQ ID NO: 19).

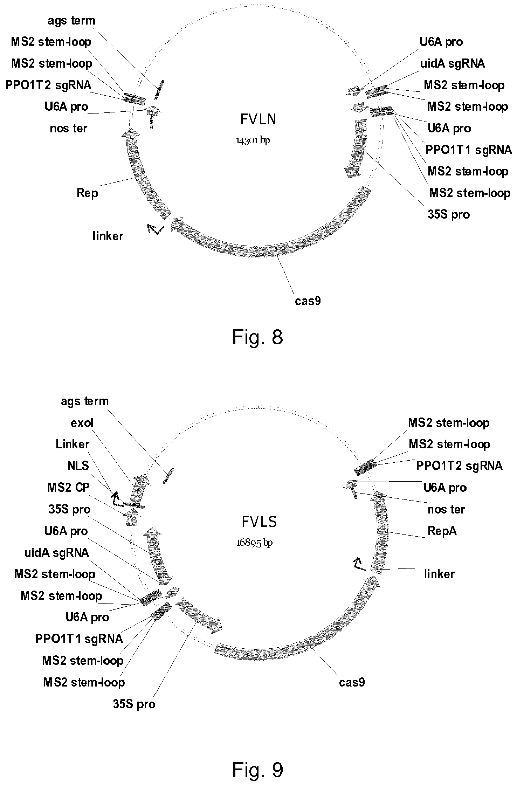

- Fig. 8: shows the FVLN vector containing a cas9 gene (SEQ ID NO: 8) translationally fused to Rep-BCTV (SEQ ID NO: 7) yielding a cas9-B-rep fusion gene (SEQ ID NO: 14). The construct also has three sgRNAs with guides for *uidA* and tobacco PPOX1 genes (SEQ ID NOs: 9-11). The *Arabidopsis* U6A promoter (SEQ ID NO: 9) was used for expression of sgRNAs containing a gene-specific guide and sgRNAs with two MS2 loops (SEQ ID NO: 19).
- Fig. 9: shows the FLVS vector containing a cassette with a cas9 gene (SEQ ID NO: 8) translationally fused to Rep-BCTV (SEQ ID NO: 7) yielding a cas9-B-rep fusion gene (SEQ ID NO: 14), and cassette with the MS2-exol fusion protein (SEQ ID NOs 19 and 15). The construct also has three sgRNAs with guides for *uidA* and tobacco PPOX1 genes (SEQ ID NOs: 9-11). The *Arabidopsis* U6A promoter (SEQ ID NO: 9) was used for expression of the sgRNAs containing gene-specific guides and sgRNAs with two MS2 loops (SEQ ID NO: 19).

### Example 1

To assess efficiency of gene targeting in tobacco a set of constructs was prepared for targeting an exogenous *uidA* gene from *E. coli.*

Transformations were carried out by infiltration (see method below)
A two base pair deletion was introduced in the *uidA* gene from *E. coli* (SEQ ID NO: 2). The modified *uidA* gene was introduced into tobacco under the cauliflower mosaic virus (CMV) 35S promoter (SEQ ID NO: 1) with a nos terminator (SEQ ID NO: 3) (ALG 492, Fig. 5). The transgenic lines were assayed for GUS activities as described by McCabe et al., (Nature Biotechnology, 1988, 6, 923-926). No GUS activity was detected in transgenic lines due to the frame shift in the *uidA* gene open reading frame.

The tobacco plants carrying the mutated *uidA* gene were then co-transformed with a repair donor comprising SEQ ID NO: 4 as part of construct FVLR (Fig. 6) and a construct expressing (i) Cas9 (construct FVLW; Fig. 7b), (ii) Cas9-Rep fusion (construct FVLN; Fig. 8) (SEQ ID NO: 14) or (iii) Cas9-Rep fusion (construct FVLS; Fig. 9) (SEQ ID NO: 14) and an exo1-MS2 fusion protein designed to bind to the sgRNA that is in turn bound to the cas9. The plants subsequently generated were assessed for GUS activity Blue sectors colour confirms repair of the *uidA* gene *in planta* using our gene targeting system (Fig. 4). Partial blue colour indicates a transformation yielding a chimeric plant. A fully blue colour indicates a fully or substantially fully transformed plant. The results of these experiments are set out in Table 1.

**Table 1**

| **Experiment** | **Chimeric plants** | **Blue plants** | **GT efficiency / blue** |
|---|---|---|---|
| (i) Donor BOR12 - PAM* uidA sgRNA2.0 35S-cas9-nos ter// PBCTV-Brep-ter | 2 out of 35 | 4 out of 35 | 11% |
| (ii) Donor BOR12 - PAM* uidA sgRNA2.0 35S-cas9-Brep-nos ter | 7 out of 59 | 19 out of 59 | 32% |
| (iii) Donor BOR12 - PAM* uidA sgRNA2.0 35S-cas9-Brep-nos ter//35S-MS2CP-Exol-ags ter | 7 out of 59 | 29 out of 59 | 49% |

These results demonstrate that gene editing mediated by the Cas9-Rep fusion (experiment (ii)) is significantly more efficient than for the control experiment using cas9 alone.

These results also demonstrate that gene editing mediated by the Cas9-Rep fusion and an exo1-MS2 fusion protein designed to bind to the Cas9-sgRNA complex (experiment (iii)) is yet more efficient than either the control experiment using cas9 alone or experiment (ii) using the Cas9-Rep fusion alone.

### Transformation Method - Infiltration

### Transformation of tobacco leaf explants with Agrobacterium strain AGLI

All items are autoclave-sterilised prior to use. Filter sterilize antibiotics to prevent fungal growth, keep antibiotics for plant tissue culture in separate box.

Sterilize plant material: take plants of about 9cm high which have not started to flower. Cut leaves having a cuticle (4-6 leaves per construct, enough to cut 100 explants), dip in 70% Ethanol and immediately dip in 1% Na-hypochlorite (use a bottle of bleach that is <3 months old because the chlorine gas evaporates), hold leaves with forceps and stir in it for 20 min. Avoid damaging the cuticle otherwise bleach will enter the vascular system. Rinse briefly in sterile water 5-6 times and leave in water until ready to be cut.

Co-cultivation of *Agrobacterium* with tobacco explants : grow AGLI in LB or L broth with appropriate antibiotics overnight at 28-30°C, the next day re-suspend *Agrobacterium* in co-cultivation solution so that the final concentration has an OD₆ₒₒₙₘ of around 0.4-0.6. Place tobacco leaves in co-culture broth and cut squares of 1-1.5cm x 1-1.5cm with a rounded sterile scalpel using a rolling action. Dip the leaf explants in the *Agrobacterium* solution with sterile forceps (stored in 100% ethanol, flamed and let to cool prior to touching the leaf tissue) blot on sterile Whatman paper and transfer on non-selective TSM plates (6 explants per plate; need to prepare about 15 plates per construct). Repeat this procedure for each construct, making sure that the scalpel and forceps are dipped in ethanol and flamed between each construct to prevent cross-contamination.

### Leave for 2 days only for AGLI (3-4 days for other Agrobacterium strains)

Transfer on selective TSM plates: use sterile flamed forceps to pick up and wash explants in 100 ml co-cultivation broth supplemented with Timentin 320mg/l (one aliquot per construct), shake well, blot on sterile Whatman paper and place the washed explants on selective TSM plates supplemented with appropriate selective antibiotics and Timentin 320mg/l to kill *Agrobacterium.*

Shoot regeneration: takes around 1 month to see shoots appear, explants should be transferred onto fresh plates every 10-14 days. Watch out for AGLI recurrent growth, if Timentin is not enough to kill *Agrobacterium,* add cefotaxime at 250mg/l.

Root regeneration: Takes around 1 week. Shoots are cut from the explants and place in growth boxes containing TRM supplemented with the appropriate selective antibiotics and Timentin 320mg/l + cefotaxime 250mg/l to prevent *Agrobacterium* recurrent growth. Maintain plants in TRM boxes: sub them every two weeks until ready to be transferred into a glasshouse.

Adaptation to glasshouse conditions: soak peat pellets in sterile water until they swell to normal size and carefully plant one plant per pellet, incubate the plants under 100% humidity conditions in a propagator, gradually opening the little windows until plants adapt to normal atmosphere over several days.

Assay the transgenic plants for GUS activities as described by McCabe et al., (Nature Biotechnology, 1988, 6, 923-926).

### Reagents:

- Co-culture: MS with vitamins and MES + O.Img/1 NAA + Img/1 BA + 3% sucrose, pH 5.7
- TSM: MS with vitamins and MES + O.Img/1 NAA + Img/1 BA + 3% sucrose, pH5.7, 0.2% gelrite
- TRM: ^ MS salts with vitamins and MES + 0.5% sucrose, pH5.7, 0.2% gelrite. Autoclave.

### Antibiotic concentrations for Agrobacterium LB or L cultures:

- To grow AGLI carrying pGreen/pSOUP: Carbenicillin IOOmg/1, Tetracycline 5mg/ml, Rifampicin 50mg/ml, Kanamycin 50mg/ml AGLI carrying pSOUP: Carbenicilin IOOmg/1, Tetracycline 5mg/ml, Rifampicin 50mg/ml. AGLI empty: Carbenicillin IOOmg/1, Rifampicin 50mg/ml.

### Antibiotic concentrations for plant culture:

- Kanamycin: 300mg/l (IOOmg/1 if using benthamiana) Hygromycin: 30mg/l (IOmg/1 if using benthamiana) PPT: 20mg/l (2mg/l if using benthamiana)
- Timentin: 320mg/l. It is used to kill agrobacterium, fairly- unstable make up small amount of stock, store in freezer for up to 1 month (after that the antibiotic is no longer efficient). Cefotaxime: 250mg/l. Also used to kill agrobacterium, add to TS.

### Nucleotide Sequences

35S promoter (SEQ ID NO: 1) 
ggaattccaatcccacaaaaatctgagcttaacagcacagttgctcctctcagagcagaatcgggtattcaacaccctcatatcaactactacgttgt
gtataacggtccacatgccggtatatacgatgactggggttgtacaaaggcggcaacaaacggcgttcccggagttgcacacaagaaatttgcca
ctattacagaggcaagagcagcagctgacgcgtacacaacaagtcagcaaacagacaggttgaacttcatccccaaaggagaagctcaactca
agcccaagagctttgctaaggccctaacaagcccaccaaagcaaaaagcccactggctcacgctaggaaccaaaaggcccagcagtgatcca
gccccaaaagagatctcctttgccccggagattacaatggacgatttcctctatctttacgatctaggaaggaagttcgaaggtgaagtagacgaca
ctatgttcaccactgataatgagaaggttagcctcttcaatttcagaaagaatgctgacccacagatggttagagaggcctacgcagcaggtctcatc
aagacgatctacccgagtaacaatctccaggagatcaaataccttcccaagaaggttaaagatgcagtcaaaagattcaggactaattgcatcaa
gaacacagagaaagacatatttctcaagatcagaagtactattccagtatggacgattcaaggcttgcttcataaaccaaggcaagtaatagagatt
ggagtctctaaaaaggtagttcctactgaatctaaggccatgcatggagtctaagattcaaatcgaggatctaacagaactcgccgtgaagactggc
gaacagttcatacagagtcttttacgactcaatgacaagaagaaaatcttcgtcaacatggtggagcacgacactctggtctactccaaaaatgtca
aagatacagtctcagaagaccaaagggctattgagacttttcaacaaaggataatttcgggaaacctcctcggattccattgcccagctatctgtcac
ttcatcgaaaggacagtagaaaaggaaggtggctcctacaaatgccatcattgcgataaaggaaaggctatcattcaagatctctctgccgacagt
ggtcccaaagatggacccccacccacgaggagcatcgtggaaaaagaagacgttccaaccacgtcttcaaagcaagtggattgatgtgacatct
ccactgacgtaagggatgacgcacaatcccactatccttcgcaagacccttcctctatataaggaagttcatttcatttggagaggacacgctcgagt
ataagagctcatttttacaacaattaccaacaacaacaaacaacaaacaacattacaattacatttacaattatcgata
uidA gene with 2bp deletion (SEQ ID NO: 2) 
atgttacgtcctgtagaaaccccaacccgtgaaatcaaaaaactcgacggcctgtgggcattcagtctggatcgcgaaaactgtggaattgatcag
cgttggtgggaaagcgcgttacaagaaagccgggcaattgctgtgccaggcagttttaacgatcagttcgccgatgcagatattcgtaattatgcgg
gcaacgtctggtatcagcgcgaagtctttataccgaaaggttgggcaggccagcgtatcgtgctgcgtttcgatgcggtcactcattacggcaaagtg
tgggtcaataatcaggaagtgatggagcatcagggcggctatacgccatttgaagccgatgtcacgccgtatgttattgccgggaaaagtacgtatc
accgtttgtgtgaacaacgaactgaactggcagactatcccgccgggaatggtgattaccgacgaaaacggcaagaaaaagcagtcttacttcca
tgatttctttaactatgccgcgatccatcgcagcgtaatgctctacaccacgccgaacacctgggtggacgatatcaccgtggtgacgcatgtcgcgc
aagactgtaaccacgcgtctgttgactggcaggtggtggccaatggtgatgtcagcgttgaactgcgtgatgcggatcaacaggtggttgcaactgg
acaaggcactagcgggactttgcaagtggtgaatccgcacctctggcaaccgggtgaaggttatctctatgaactgtgcgtcacagccaaaagcca
gacagagtgtgatatctacccgcttcgcgtcggcatccggtcagtggcagtgaagggcgaacagttcctgattaaccacaaaccgttctactttactg
gctttggtcgtcatgaagatgcggacttacgtggcaaaggattcgataacgtgctgatggtgcacgaccacgcattaatggactggattggggccaa
ctcctaccgtacctcgcattacccttacgctgaagagatgctcgactgggcagatgaacatggcatcgtggtgattgatgaaactgctgctgtcggcttt
aacctctctttaggcattggtttcgaagcgggcaacaagccgaaagaactgtacagcgaagaggcagtcaacggggaaactcagcaagcgcac
ttacaggcgattaaagagctgatagcgcgtgacaaaaaccacccaagcgtggtgatgtggagtattgccaacgaaccggatacccgtccgcaag
tgcacgggaatatttcgccactggcggaagcaacgcgtaaactcgacccgacgcgtccgatcacctgcgtcaatgtaatgttctgcgacgctcaca
ccgataccatcagcgatctctttgatgtgctgtgcctgaaccgttattacggatggtatgtccaaagcggcgatttggaaacggcagagaaggtactg
gaaaaagaacttctggcctggcaggagaaactgcatcagccgattatcatcaccgaatacggcgtggatacgttagccgggctgcactcaatgta
caccgacatgtggagtgaagagtatcagtgtgcatggctggatatgtatcaccgcgtctttgatcgcgtcagcgccgtcgtcggtgaacaggtatgga
atttcgccgattttgcgacctcgcaaggcatattgcgcgttggcggtaacaagaaagggatcttcactcgcgaccgcaaaccgaagtcggcggctttt
ctgctgcaaaaacgctggactggcatgaacttcggtgaaaaaccgcagcagggaggcaaacaatga
nos terminator (SEQ ID NO: 3 gtcgaagcagatcgttcaaacatttggcaataaagtttcttaagattgaatcctgttgccggtcttgcgatgattatcatataatttctgttgaattacgttaa gcatgtaataattaacatgtaatgcatgacgttatttatgagatgggtttttatgattagagtcccgcaattatacatttaatacgcgatagaaaacaaaat atagcgcgcaaactaggataaattatcgcgcgcggtgtcatctatgttactagatcgac
uidA donor (SEQ ID NO: 4) 
cgtcctgtagaaaccccaacccgtgaaatcaaaaaactcgacggcctgtgggcattcagtctggatcgcgaaaactgtggaattgatcagcgttgg
tgggaaagcgcgttacaagaaagccgggcaattgctgtgccaggcagttttaacgatcagttcgccgatgcagatattcgtaattatgcgggcaacg
tctggtatcagcgcgaagtctttataccgaaaggttgggcaggccagcgtatcgtgctgcgtttcgatgcggtcactcattacggcaaagtgtgggtca
ataatcaggaagtgatggagcatcagggcggctatacgccatttgaagccgatgtcacgccgtatgttattgccgggaaaagtgtacgtatcactgtt
tgtgtgaacaacgaactgaactggcagactatcccgccgggaatggtgattaccgacgaaaacggcaagaaaaagcagtcttacttccatgatttc
tttaactatgccggaatccatcgcagcgtaatgctctacaccacgccgaacacctgggtggacgatatcaccgtggtgacgcatgtcgcgcaagac
tgtaaccacgcgtctgttgactggcaggtggtggccaatggtgatgtcagcgttgaactgcgtgatgcggatcaacaggtggttgcaactggacaag
gcactagcgggactttgcaagtggtgaatccgcacctctggcaaccgggtgaaggttatctctatgaactgtgcgtcacagccaaaagccagacag
agtgtgatatctacccgcttcgcgtcggcatccggtcagtggcagtgaagggcgaacagttcctgattaaccacaaaccgttctactttactggctttgg
 
tcgtcatgaagatgcggacttgcgtggcaaaggattcgataacgtgctgatggtgcacgaccacgcattaatggactggattggggccaactcctac
cgtacctcgcattacccttacgctgaagagatgctcgactgggcagatgaacatggcatcgtggtgattgatgaaactgctgctgtcggctttaacctct
ctttaggcattggtttcgaagcgggcaacaagccgaaagaactgtacagcgaagaggcagtcaacggggaaactcagcaagcgcacttacagg
cgattaaagagctgatagcgcgtgacaaaaaccacccaagcgtggtgatgtggagtattgccaacgaaccggatacccgtccgcaaggtgcac
gggaatatttcgcgccactggcggaagcaacgcgtaaactcgacccgacgcgtccgatcacctgcgtcaatgtaatgttctgcgacgctcacaccg
ataccatcagcgatctctttgatgtgctgtgcctgaaccgttattacggatggtatgtccaaagcggcgatttggaaacggcagagaaggtactggaa
aaagaacttctggcctggcaggagaaactgcatcagccgattatcatcaccga
tobacco PPOX1 donor (SEQ ID NO: 5) 
tgctgccggatgcaatcagtgcaaggtgcccttctcctttcccagttcaatgtctccaccccactcccacccccagtataaaattcagggggaaaa
agagaagaaaaatatcaaaatttaaggactagttaaagttcagttttggcatttttctgcctaagtgcaatattatcaaaggtgaaaagcgcaaaaaa
gctctaaagtctattggggctttaagcgcaaagcgcaaataaagcatgggctttaatgcagaaaggctcaaatgaaaatgaaaagaaaaagaaa
actacaaatatgtatgtatagtccaagactaatatctataagcatgaataccaaatatatggacaaagacattgaagaaactttattataaagtgaaat
atcaattgtttagcatcgcctcttcaggattacgcttattggcaaggaaaagtatgctttagagccttgatgacaacgctgaagcgcccgctaagcga
ggcaaagcgctcaacatgttttgagcctcgcttcagggcttaagcgttatcaaagtgtgttctttggtgcccttgttcaagcagttctctagtatttttaccttg
gctccaccccactcccacccccctgtataaacgtttagggggggaaaagaaagagaagaaaaattatcaaaagttaagaaagctaagttttggctttt
ttctgctaggtgtgttctttggtgctcttgttcaagcagttctctagtatttttaccttggaaatgctaataagttgtcgccgtgtttccttagattgggaagcaaa
ttaaaactatcatggaagcttcttagcattactaagtcagaaaaagcaggatatcgcttgacatacgagacaccagaaggagtagtttctcttcaaag
tcgaagcattgtcatgactgtgccatcctatgtagcaagcaacatattacgtcctctttcggtgtgtttccattactgtttgctccaaagtcgttattgctattgt
tcatttggcactctaatatagaaacttggataaaaaagggggatcctttaatttttctttttcatttggactaggaaaagcggctcaaacacatgtataacag
tcatatactgtgtataatatgtgtatgcaatataatgtgcagagaaagacttatcggagagaagagaattaaaaaaaagaacatttgtgtcacacgga
gaattaacatgtacatgcttaatgaggtcaagagcgcaaccgtatcccacccctcatgaaagattgaacctgtatctcatgttattgtgagctttttcata
tataaatttatgagcaattttctttaaatgctttgaccttggatttttcgaaggttactacttctgataattgctttgcaagaaatgcggctcacttaaaatggga
aacgtttatctataggttgtggttcagcttcttctgtttcttgctaaagcaagatgtctcctaaacttatattaaactcaggttgccgcagcagatgcactttca
aatttctactatcccccagttggagcagtcacaatttcatatcctcaagaagctattcgtgatgaacgtctggttgatggtgaactaaagggatttgggca
gttgcatccacgtacacagggagtggaaacactaggtagctttctgtttgaaggattcactctaattttaacttcaagaatccatattgaagatggaaaa
cttttattacgacaattgcaagatgcaactcactgaagtagcattcctctattgcatctgcaggaataatatatagcgtatcagtcttccctgaccatcatt
acctagaaattttgacagtaccatgcaaatcgaagatgaatgagttcagaacaataacatcaggatttgattcatcttaaatataatacttgcgtttcata
tgcaggaacgatatatagttcatcactcttccctaaccgtgccccaaaaggtcgggtgctactcttgaacatgattgcaggagcaaaaaatccggaa
attttgtctaaggtaaggcatttaaattgagcgcaacttcaagtcctgccggtattgatttatcataatgaatattcatgggctggccagttggggcatcgc
ttgaaggttcctatggtaacaaacagaaaactgagaatgaccttatcccctgatctcttttggttgcaaatacgttaaccttgcagtgatgaggagaatg
ttttcagtgttattttctctagattaatggcgtagttggtttggattacaaaaccgtttaggcttatatgtgattagagtctccaggtttttgatctagcagtaaga
gcaaaatgtgtgatgttttaaaaaaacagtcaagtgattacaaatactcagaatagtttcaccataaagaactaaagagctaagaagattgtggtttct
ctttcctctatgttcgactgcatttgtgggttttccatagcatgactttcttttatggtacttgtacgaaagcatttcgctgttattctttatgcttcagggcctaga
ataaattgctttgagatttgatccctcattcttcttgcagacggagagccaacttgtggaagtagttgatcgtgacctcagaaaaatgcttatagaaccca
aagcacaagatccccttgttgtgggtgtgcgagtatggccac
viral origin of replication (BOR) from Beet Curly Top Virus (BCTV) (SEQ ID NO: 6) 
tcctgtactccgatgacgtggcttagcatattaacatatctattggagtattggagtattatatatattagtacaactttcataagggccatccgttataatatt
accggatggcccgaaaaaaatgggcacccaatcaaaacgtgacacgtggaaggggactgttgaatgatgtgacgtttttgagcgggaaacttcct
gaag
BCTV rep gene (SEQ ID NO: 7) 
atgcctcctactaaaagatttcgtattcaagcaaaaaacatatttcttacatatcctcagtgttctctttcaaaagaagaagctcttgagcaaattcaaag
aatacaactttcatctaataaaaaatatattaaaattgccagagagctacacgaagatgggcaacctcatctccacgtcctgcttcaactcgaagga
aaagttcagatcacaaatatcagattattcgacctggtatccccaaccaggtcagcacatttccatccaaacattcagagagctaaatccagctccg
acgtcaagtcctacgtagacaaggacggagacacaattgaatggggagaattccagatcgacggtagaagtgctagaggaggtcaacagaca
gctaacgactcatatgccaaggcgttaaacgcaacttctcttgaccaagcacttcaaatattgaaggaagaacaaccaaaggattacttccttcaac
atcacaatcttttgaacaatgctcaaaagatatttcagaggccacctgatccatggactccactatttcctctgtcctcattcacaaacgttcctgaggaa
atgcaagaatgggctgatgcatatttcggggttgatgccgctgcgcggcctttaagatataatagtatcatagtagagggtgattcaagaacaggga
agactatgtgggctagatctttaggggcccacaattacatcacagggcacttagattttagccctagaacgtattatgatgaagtggaatacaacgtc
attgatgacgtagatcccacttacttaaagatgaaacactggaaacaccttattggagcacaaaaggagtggcagacaaacttaaagtatggaaa
accacgtgtcattaaaggtggtatcccctgcattatattatgcaatccaggacctgagagctcataccaacaatttcttgaaaaaccagaaaatgaag
cccttaagtcctggacattacataattcaaccttctgcaaactccaaggtccgctctttaataaccaagcagcagcatcctcgcaaggtgactctaccc
tgtaactgccacttcacaatacaccatgaatgtaatagaggattttcgcacaggggaacctattactctccatcaggcaacaaattccgtcgaattcga
gaatgtaccgaatccactgtatatgaaactcctatggttcgagagatacgggccaatctatcaactgaagatacaaatcagattcaactacaacctc
cggagagcgttgaatcttcacaagtgctggatagagctgacgataactggatcgaacaggatattgactggaccccgtttcttgaaggtcttgaaaa
agagactagagatatacttggataatctaggtttaatttgtattaataatgtaattagaggtttaaatcatgtcctgtatgaagaatttacttttgtatcaagtg
taattcagaaccagagtgttgcaatgaacttgtactaatttcattattaataataaatattattaataaaaatagcatctacaattgccaaataatgtggca
tacatattagtattatccgtattatcattaacaacaacatagagtaaagcattctccttcacgtcttcatacttccc
cas9 gene (SEQ ID NO: 8) 
atggacaagaagtacagcatcggcctggacatcggcacgaactcggtgggctgggcggtgatcacggacgagtacaaggtgccctccaagaa
gttcaaggtgctgggcaacaccgaccgccactcgatcaagaagaacctgatcggcgccctgctgttcgactccggcgagaccgccgaggcgac
gcgcctgaagcgcaccgcgcgtcgccgctacacgcgtcgcaagaaccgcatctgctacctgcaggagatcttcagcaacgagatggccaaggt
ggacgactcgttcttccaccgcctggaggagtccttcctggtggaggaagacaagaagcacgagcgccaccccatcttcggcaacatcgtggacg
aggtggcctaccacgagaagtacccgacgatctaccacctgcgcaagaagctggtggacagcaccgacaaggcggacctgcgcctgatctacc
tggccctggcgcacatgatcaagttccgcggccacttcctgatcgagggcgacctgaaccccgacaactcggacgtggacaagctgttcatccag
ctggtgcagacctacaaccagctgttcgaggagaacccgatcaacgcctccggcgtggacgccaaggcgatcctgagcgcgcgcctgtccaag
agccgtcgcctggagaacctgatcgcccagctgcccggcgagaagaagaacggcctgttcggcaacctgatcgcgctgtcgctgggcctgacgc
cgaacttcaagtccaacttcgacctggccgaggacgcgaagctgcagctgagcaaggacacctacgacgacgacctggacaacctgctggccc
agatcggcgaccagtacgcggacctgttcctggccgcgaagaacctgtcggacgccatcctgctgtccgacatcctgcgcgtgaacaccgagatc
acgaaggcccccctgtcggcgtccatgatcaagcgctacgacgagcaccaccaggacctgaccctgctgaaggcgctggtgcgccagcagctg
ccggagaagtacaaggagatcttcttcgaccagagcaagaacggctacgccggctacatcgacggcggcgcgtcgcaagaggagttctacaag
ttcatcaagcccatcctggagaagatggacggcacggaggagctgctggtgaagctgaaccgcgaggacctgctgcgcaagcagcgcaccttc
gacaacggcagcatcccccaccagatccacctgggcgagctgcacgccatcctgcgtcgccaagaggacttctacccgttcctgaaggacaacc
gcgagaagatcgagaagatcctgacgttccgcatcccctactacgtgggcccgctggcccgcggcaacagccgcttcgcgtggatgacccgcaa
gtcggaggagaccatcacgccctggaacttcgaggaagtggtggacaagggcgccagcgcgcagtcgttcatcgagcgcatgaccaacttcga
caagaacctgcccaacgagaaggtgctgccgaagcactccctgctgtacgagtacttcaccgtgtacaacgagctgacgaaggtgaagtacgtg
accgagggcatgcgcaagcccgccttcctgagcggcgagcagaagaaggcgatcgtggacctgctgttcaagaccaaccgcaaggtgacggt
gaagcagctgaaagaggactacttcaagaagatcgagtgcttcgacagcgtggagatctcgggcgtggaggaccgcttcaacgccagcctggg
cacctaccacgacctgctgaagatcatcaaggacaaggacttcctggacaacgaggagaacgaggacatcctggaggacatcgtgctgaccct
gacgctgttcgaggaccgcgagatgatcgaggagcgcctgaagacgtacgcccacctgttcgacgacaaggtgatgaagcagctgaagcgtcg
ccgctacaccggctggggccgcctgagccgcaagctgatcaacggcatccgcgacaagcagtccggcaagaccatcctggacttcctgaagag
cgacggcttcgcgaaccgcaacttcatgcagctgatccacgacgactcgctgaccttcaaagaggacatccagaaggcccaggtgtcgggccag
ggcgactccctgcacgagcacatcgccaacctggcgggctcccccgcgatcaagaagggcatcctgcagaccgtgaaggtggtggacgagctg
gtgaaggtgatgggccgccacaagccggagaacatcgtgatcgagatggcccgcgagaaccagaccacgcagaagggccagaagaacag
ccgcgagcgcatgaagcgcatcgaggaaggcatcaaggagctgggctcgcagatcctgaaggagcaccccgtggagaacacccagctgcag
aacgagaagctgtacctgtactacctgcagaacggccgcgacatgtacgtggaccaggagctggacatcaaccgcctgtccgactacgacgtgg
accacatcgtgccccagagcttcctgaaggacgactcgatcgacaacaaggtgctgacccgcagcgacaagaaccgcggcaagagcgacaa
cgtgccgtcggaggaagtggtgaagaagatgaagaactactggcgccagctgctgaacgccaagctgatcacgcagcgcaagttcgacaacct
gaccaaggccgagcgcggtggcctgtcggagctggacaaggcgggcttcatcaagcgccagctggtggagacccgccagatcacgaagcac
gtggcgcagatcctggactcccgcatgaacacgaagtacgacgagaacgacaagctgatccgcgaggtgaaggtgatcaccctgaagtccaag
ctggtcagcgacttccgcaaggacttccagttctacaaggtgcgcgagatcaacaactaccaccacgcccacgacgcgtacctgaacgccgtggt
gggcaccgcgctgatcaagaagtaccccaagctggagagcgagttcgtgtacggcgactacaaggtgtacgacgtgcgcaagatgatcgccaa
gtcggagcaggagatcggcaaggccaccgcgaagtacttcttctactccaacatcatgaacttcttcaagaccgagatcacgctggccaacggcg
agatccgcaagcgcccgctgatcgagaccaacggcgagacgggcgagatcgtgtgggacaagggccgcgacttcgcgaccgtgcgcaaggt
gctgagcatgccccaggtgaacatcgtgaagaagaccgaggtgcagacgggcggcttctccaaggagagcatcctgccgaagcgcaactcgg
acaagctgatcgcccgcaagaaggactgggaccccaagaagtacggcggcttcgactccccgaccgtggcctacagcgtgctggtggtggcga
aggtggagaagggcaagtccaagaagctgaagagcgtgaaggagctgctgggcatcaccatcatggagcgcagctcgttcgagaagaacccc
atcgacttcctggaggccaagggctacaaagaggtgaagaaggacctgatcatcaagctgccgaagtactcgctgttcgagctggagaacggcc
gcaagcgcatgctggcctccgcgggcgagctgcagaagggcaacgagctggccctgcccagcaagtacgtgaacttcctgtacctggcgtccca
ctacgagaagctgaagggctcgccggaggacaacgagcagaagcagctgttcgtggagcagcacaagcactacctggacgagatcatcgag
cagatctcggagttctccaagcgcgtgatcctggccgacgcgaacctggacaaggtgctgagcgcctacaacaagcaccgcgacaagcccatc
cgcgagcaggcggagaacatcatccacctgttcaccctgacgaacctgggcgccccggccgcgttcaagtacttcgacaccacgatcgaccgca
agcgctacacctccacgaaagaggtgctggacgcgaccctgatccaccagagcatcaccggcctgtacgagacgcgcatcgacctgagccag
ctgggcggcgactcccgcgcggacccgaagaagaagcgcaaggtgtaa
Arabidopsis U6A promoter (SEQ ID NO: 9) 
catcttcattcttaagatatgaagataatcttcaaaaggcccctgggaatctgaaagaagagaagcaggcccatttatatgggaaagaacaatagta
tttcttatataggcccatttaagttgaaaacaatcttcaaaagtcccacatcgcttagataagaaaacgaagctgagtttatatacagctagagtcgaa
gtagt
sgRNA2.0 with MS2 aptamer hairpins for uidA gene (SEQ ID NO: 10) 
cagttcgttgttcacacaagttttagagctaggccaacatgaggatcacccatgtctgcagggcctagcaagttaaaataaggctagtccgttatcaa
cttggccaacatgaggatcacccatgtctgcagggccaagtggcaccgagtcggtgcttttttttttt
sgRNA2.0-1 for tobacco PPOX1 gene (SEQ ID NO: 11) 
attactaagtcagaaaagttttagagctaggccaacatgaggatcacccatgtctgcagggcctagcaagttaaaataaggctagtccgttatcaact
tggccaacatgaggatcacccatgtctgcagggccaagtggcaccgagtcggtgcttttttttttt
sgRNA2.0-2 for tobacco PPOX1 gene (SEQ ID NO: 12) 
tgctactcttgaactacatggttttagagctaggccaacatgaggatcacccatgtctgcagggcctagcaagttaaaataaggctagtccgttatcaa
cttggccaacatgaggatcacccatgtctgcagggccaagtggcaccgagtcggtgcttttttttt
ags terminator (SEQ ID NO: 13) 
gaattaacagaggtggatggacagacccgttcttacaccggactgggcgcgggataggatattcagattgggatgggattgagcttaaagccggc
gctgagaccatgctcaaggtaggcaatgtcctcagcgtcgagcccggcatctatgtcgagggcattggtggagcgcgcttcggggataccgtgcttg
taactgagaccggatatgaggccctcactccgcttgatcttggcaaagatatttgacgcatttattagtatgtgttaattttcatttgcagtgcagtattttcta
ttcgatctttatgtaattcgttacaattaataaatattcaaatcagattattgactgtcatttgtatcaaatcgtgtttaatggatatttttattataatattgatgat
cas9-B-rep fusion gene (SEQ ID NO: 14) 
atggacaagaagtacagcatcggcctggacatcggcacgaactcggtgggctgggcggtgatcacggacgagtacaaggtgccctccaagaa
gttcaaggtgctgggcaacaccgaccgccactcgatcaagaagaacctgatcggcgccctgctgttcgactccggcgagaccgccgaggcgac
gcgcctgaagcgcaccgcgcgtcgccgctacacgcgtcgcaagaaccgcatctgctacctgcaggagatcttcagcaacgagatggccaaggt
ggacgactcgttcttccaccgcctggaggagtccttcctggtggaggaagacaagaagcacgagcgccaccccatcttcggcaacatcgtggacg
aggtggcctaccacgagaagtacccgacgatctaccacctgcgcaagaagctggtggacagcaccgacaaggcggacctgcgcctgatctacc
tggccctggcgcacatgatcaagttccgcggccacttcctgatcgagggcgacctgaaccccgacaactcggacgtggacaagctgttcatccag
ctggtgcagacctacaaccagctgttcgaggagaacccgatcaacgcctccggcgtggacgccaaggcgatcctgagcgcgcgcctgtccaag
agccgtcgcctggagaacctgatcgcccagctgcccggcgagaagaagaacggcctgttcggcaacctgatcgcgctgtcgctgggcctgacgc
cgaacttcaagtccaacttcgacctggccgaggacgcgaagctgcagctgagcaaggacacctacgacgacgacctggacaacctgctggccc
agatcggcgaccagtacgcggacctgttcctggccgcgaagaacctgtcggacgccatcctgctgtccgacatcctgcgcgtgaacaccgagatc
acgaaggcccccctgtcggcgtccatgatcaagcgctacgacgagcaccaccaggacctgaccctgctgaaggcgctggtgcgccagcagctg
ccggagaagtacaaggagatcttcttcgaccagagcaagaacggctacgccggctacatcgacggcggcgcgtcgcaagaggagttctacaag
ttcatcaagcccatcctggagaagatggacggcacggaggagctgctggtgaagctgaaccgcgaggacctgctgcgcaagcagcgcaccttc
gacaacggcagcatcccccaccagatccacctgggcgagctgcacgccatcctgcgtcgccaagaggacttctacccgttcctgaaggacaacc
gcgagaagatcgagaagatcctgacgttccgcatcccctactacgtgggcccgctggcccgcggcaacagccgcttcgcgtggatgacccgcaa
gtcggaggagaccatcacgccctggaacttcgaggaagtggtggacaagggcgccagcgcgcagtcgttcatcgagcgcatgaccaacttcga
caagaacctgcccaacgagaaggtgctgccgaagcactccctgctgtacgagtacttcaccgtgtacaacgagctgacgaaggtgaagtacgtg
accgagggcatgcgcaagcccgccttcctgagcggcgagcagaagaaggcgatcgtggacctgctgttcaagaccaaccgcaaggtgacggt
gaagcagctgaaagaggactacttcaagaagatcgagtgcttcgacagcgtggagatctcgggcgtggaggaccgcttcaacgccagcctggg
cacctaccacgacctgctgaagatcatcaaggacaaggacttcctggacaacgaggagaacgaggacatcctggaggacatcgtgctgaccct
gacgctgttcgaggaccgcgagatgatcgaggagcgcctgaagacgtacgcccacctgttcgacgacaaggtgatgaagcagctgaagcgtcg
ccgctacaccggctggggccgcctgagccgcaagctgatcaacggcatccgcgacaagcagtccggcaagaccatcctggacttcctgaagag
cgacggcttcgcgaaccgcaacttcatgcagctgatccacgacgactcgctgaccttcaaagaggacatccagaaggcccaggtgtcgggccag
ggcgactccctgcacgagcacatcgccaacctggcgggctcccccgcgatcaagaagggcatcctgcagaccgtgaaggtggtggacgagctg
gtgaaggtgatgggccgccacaagccggagaacatcgtgatcgagatggcccgcgagaaccagaccacgcagaagggccagaagaacag
ccgcgagcgcatgaagcgcatcgaggaaggcatcaaggagctgggctcgcagatcctgaaggagcaccccgtggagaacacccagctgcag
aacgagaagctttacctgtactacctgcagaacggccgcgacatgtacgtggaccaggagctggacatcaaccgcctgtccgactacgacgtgg
accacatcgtgccccagagcttcctgaaggacgactcgatcgacaacaaggtgctgacccgcagcgacaagaaccgcggcaagagcgacaa
cgtgccgtcggaggaagtggtgaagaagatgaagaactactggcgccagctgctgaacgccaagctgatcacgcagcgcaagttcgacaacct
gaccaaggccgagcgcggtggcctgtcggagctggacaaggcgggcttcatcaagcgccagctggtggagacccgccagatcacgaagcac
gtggcgcagatcctggactcccgcatgaacacgaagtacgacgagaacgacaagctgatccgcgaggtgaaggtgatcaccctgaagtccaag
ctggtcagcgacttccgcaaggacttccagttctacaaggtgcgcgagatcaacaactaccaccacgcccacgacgcgtacctgaacgccgtggt
gggcaccgcgctgatcaagaagtaccccaagctggagagcgagttcgtgtacggcgactacaaggtgtacgacgtgcgcaagatgatcgccaa
gtcggagcaggagatcggcaaggccaccgcgaagtacttcttctactccaacatcatgaacttcttcaagaccgagatcacgctggccaacggcg
agatccgcaagcgcccgctgatcgagaccaacggcgagacgggcgagatcgtgtgggacaagggccgcgacttcgcgaccgtgcgcaaggt
gctgagcatgccccaggtgaacatcgtgaagaagaccgaggtgcagacgggcggcttctccaaggagagcatcctgccgaagcgcaactcgg
acaagctgatcgcccgcaagaaggactgggaccccaagaagtacggcggcttcgactccccgaccgtggcctacagcgtgctggtggtggcga
aggtggagaagggcaagtccaagaagctgaagagcgtgaaggagctgctgggcatcaccatcatggagcgcagctcgttcgagaagaacccc
atcgacttcctggaggccaagggctacaaagaggtgaagaaggacctgatcatcaagctgccgaagtactcgctgttcgagctggagaacggcc
gcaagcgcatgctggcctccgcgggcgagctgcagaagggcaacgagctggccctgcccagcaagtacgtgaacttcctgtacctggcgtccca
ctacgagaagctgaagggctcgccggaggacaacgagcagaagcagctgttcgtggagcagcacaagcactacctggacgagatcatcgag
cagatctcggagttctccaagcgcgtgatcctggccgacgcgaacctggacaaggtgctgagcgcctacaacaagcaccgcgacaagcccatc
cgcgagcaggcggagaacatcatccacctgttcaccctgacgaacctgggcgccccggccgcgttcaagtacttcgacaccacgatcgaccgca
agcgctacacctccacgaaagaggtgctggacgcgaccctgatccaccagagcatcaccggcctgtacgagacgcgcatcgacctgagccag
ctgggcggcgactcccgcgcggacccgaagaagaagcgcaaggtgagcgctggaggaggtggaagcggaggaggaggaagcggaggag
gaggtagcggatccatgcctcctactaaaagatttcgtattcaagcaaaaaacatatttcttacatatcctcagtgttctctttcaaaagaagaagctctt
gagcaaattcaaagaatacaactttcatctaataaaaaatatattaaaattgccagagagctacacgaagatgggcaacctcatctccacgtcctgc
ttcaactcgaaggaaaagttcagatcacaaatatcagattattcgacctggtatccccaaccaggtaattttcatctttgtttggccttccaagtgcttttttt
gctgtttacgggtggaacttcagtaaaaatgggatcaaaacatcatatggcataaataaattttaagaatggcgaactcggggttaccgaatatggct
tcctttttcagtgtttcttagtccattgtacttatgagattgcaggtcagcacatttccatccaaacattcagagagctaaatccagctccgacgtcaagtcc
tacgtagacaaggacggagacacaattgaatggggagaattccagatcgacggtagaagtgctagaggaggtcaacagacagctaacgactc
atatgccaaggcgttaaacgcaacttctcttgaccaagcacttcaaatattgaaggaagaacaaccaaaggattacttccttcaacatcacaatctttt
gaacaatgctcaaaagatatttcagaggccacctgatccatggactccactatttcctctgtcctcattcacaaacgttcctgaggaaatgcaagaatg
ggctgatgcatatttcggggttgatgccgctgcgcggcctttaagatataatagtatcatagtagagggtgattcaagaacaggaagactatgtggg
ctagatctttaggggcccacaattacatcacagggcacttagatttagccctagaacgtattatgatgaagtggaatacaacgtcattgatgacgtag
 
atcccacttacttaaagatgaaacactggaaacaccttattggagcacaaaaggagtggcagacaaacttaaagtatggaaaccacgtgtcatt
aaaggtggtatcccctgcattatattatgcaatccaggacctgagagcacataccaacaatttcttgaaaaaccagaaaatgaagcccttaagtcct
ggacattacataattcaaccttctgcaaactccaaggtccgctctttaataaccaagcagcagcatcctcgcaaggtgactctaccctgtaactgcca
cttcacaatacaccatgaatgtaatagaggattttcgcacaggggaacctattactctccatcaggcaacaaattccgtcgaattcgagaatgtaccg
aatccactgtatatgaaactcctatggttcgagagatacgggccaatctatcaactgaagatacaaatcagattcaactacaacctccggagagcgt
tgaatcttcacaagtgctggatagagctgacgataactggatcgaacaggatattgactggaccccgtttcttgaaggtaagaaatcttttcccatcttg
aagtcacctcaaaccgaacgttaggaaattccaaaatgtttgatagtagtctacttagtttcaagttttgggtttgtgtatactttcactaataatatgcgtg
gaaacattgcaggtcttgaaaaagagactagagatatacttggataatctaggtttaatttgtattaataatgtaattagaggtttaaatcatgtcctgtat
gaagaatttacttttgtatcaagtgtaattcagaaccagagtgttgcaatgaacttgtactaa
exol gene from bacteriophage λ (SEQ ID NO: 15) 
atgacaccggacattatcctgcagcgtaccgggatcgatgtgagagctgtcgaacagggggatgatgcgtggcacaaattacggctcggcgtcat
caccgcttcagaagttcacaacgtgatagcaaaaccccgctccggaaagaagtggcctgacatgaaaatgtcctacttccacaccctgcttgctga
ggtttgcaccggtgtggctccggaagttaacgctaaagcactggcctggggaaaacagtacgagaacgacgccagaaccctgtttgaattcacttc
cggcgtgaatgttactgaatccccgatcatctatcgcgacgaaagtatgcgtaccgcctgctctcccgatggtttatgcagtgacggcaacggccttg
aactgaaatgcccgtttacctcccgggatttcatgaagttccggctcggtggtttcgaggccataaagtcagcttacatggcccaggtgcagtacagc
atgtgggtgacgcgaaaaaatgcctggtactttgccaactatgacccgcgtatgaagcgtgaaggcctgcattatgtcgtgattgagcggatgaaa
agtacatggcgagttttgacgagatcgtgccggagttcatcgaaaaaatggacgaggcactggctgaaattggttttgtatttggggagcaatggcg
atga
sgRNA comprising multiple stem-loops (SEQ ID NO: 16) 
gttttagagctaggccaacatgaggatcacccatgtctgcagggcctagcaagttaaaataaggctagtccgttatcaactttaaggagtttatatgga
aacccttaaagtggcaccgagtcggtgctaccgccgacaacgcggtttttttttt
triple fusion of E.coli recA gene (SEQ ID NO: 17) 
atggacgagaacaagaagcgcgccctggccgcggccctgggacagatcgaacgccaattcggcaaaggcgcggtcatgcgcatgggcgacc
atgagcgccaggcgatcccggccatctccaccggctccctgggtctggacatcgccctcggcatcggcggcctgcccaagggccggatcgtcga
gatctacggtccggaatcctcgggcaagaccaccctgaccctctcggtgatcgccgaggcccagaaacagggcgccacctgtgccttcgtcgacg
ccgagcacgcgctcgatcccgactatgccggcaagctgggcgtcaacgtcgacgacctgctggtctcccagccggacaccggcgagcaggccc
tggaaatcaccgacatgctggtgcgctccaacgcggtcgacgtgatcatcgtcgactccgtggccgcgctggtacccaaggccgagatcgaaggc
gagatgggcgacgcccacgtcggcctgcaggcacgcctgatgtcccaggcgctgcgcaagatcaccggcaatatcaagaacgccaactgcctg
gtcatcttcatcaaccagatccgcatgaagatcggcgtcatgttcggcaacccggaaaccaccaccggcggtaacgcactgaagttctacgcctcg
gtccgcctggacatccgtcgtaccggcgcggtgaaggaaggcgacgaggtggtgggtagcgaaacccgcgtcaaggtggtgaagaacaaggtt
tccccgccgttccgccaggccgagttccagatcctctacggtaagggcatctaccgtaccggcgagatcatcgatctgggcgtgcaattgggcctgg
tcgagaagtccggcgcctggtacagctaccagggcagcaagatcggccagggcaaggcgaacgccgccaagtacctggaagacaatccgga
aatcggttcggtactggagaagaccattcgcgaccagttgctggccaagagcggcccggtgaaggccgacgccgaagaagtggctgacgccga
agccgattcagagctcggagaaggtcaaggacagggacaaggtccaggacgaggatacgcatataagcttgacgagaacaagaagcgcgcc
ctggccgcggccctgggacagatcgaacgccaattcggcaaaggcgcggtcatgcgcatgggcgaccatgagcgccaggcgatcccggccat
ctccaccggctccctgggtctggacatcgccctcggcatcggcggcctgcccaagggccggatcgtcgagatctacggtccggaatcctcgggca
agaccaccctgaccctctcggtgatcgccgaggcccagaaacagggcgccacctgtgccttcgtcgacgccgagcacgcgctcgatcccgacta
tgccggcaagctgggcgtcaacgtcgacgacctgctggtctcccagccggacaccggcgagcaggccctggaaatcaccgacatgctggtgcg
ctccaacgcggtcgacgtgatcatcgtcgactccgtggccgcgctggtacccaaggccgagatcgaaggcgagatgggcgacgcccacgtcgg
cctgcaggcacgcctgatgtcccaggcgctgcgcaagatcaccggcaatatcaagaacgccaactgcctggtcatcttcatcaaccagatccgca
tgaagatcggcgtcatgttcggcaacccggaaaccaccaccggcggtaacgcactgaagttctacgcctcggtccgcctggacatccgtcgtacc
ggcgcggtgaaggaaggcgacgaggtggtgggtagcgaaacccgcgtcaaggtggtgaagaacaaggtttccccgccgttccgccaggccga
gttccagatcctctacggtaagggcatctaccgtaccggcgagatcatcgatctgggcgtgcaattgggcctggtcgagaagtccggcgcctggtac
agctaccagggcagcaagatcggccagggcaaggcgaacgccgccaagtacctggaagacaatccggaaatcggttcggtactggagaaga
ccattcgcgaccagttgctggccaagagcggcccggtgaaggccgacgccgaagaagtggctgacgccgaagccgattcaggatccggagaa
ggtcaaggacagggacaaggtccaggacgaggatacgcatatgcatgcgacgagaacaagaagcgcgccctggccgcggccctgggacag
atcgaacgccaattcggcaaaggcgcggtcatgcgcatgggcgaccatgagcgccaggcgatcccggccatctccaccggctccctgggtctgg
acatcgccctcggcatcggcggcctgcccaagggccggatcgtcgagatctacggtccggaatcctcgggcaagaccaccctgaccctctcggtg
atcgccgaggcccagaaacagggcgccacctgtgccttcgtcgacgccgagcacgcgctcgatcccgactatgccggcaagctgggcgtcaac
gtcgacgacctgctggtctcccagccggacaccggcgagcaggccctggaaatcaccgacatgctggtgcgctccaacgcggtcgacgtgatca
tcgtcgactccgtggccgcgctggtacccaaggccgagatcgaaggcgagatgggcgacgcccacgtcggcctgcaggcacgcctgatgtccc
aggcgctgcgcaagatcaccggcaatatcaagaacgccaactgcctggtcatcttcatcaaccagatccgcatgaagatcggcgtcatgttcggc
aacccggaaaccaccaccggcggtaacgcactgaagttctacgcctcggtccgcctggacatccgtcgtaccggcgcggtgaaggaaggcgac
gaggtggtgggtagcgaaacccgcgtcaaggtggtgaagaacaaggtttccccgccgttccgccaggccgagttccagatcctctacggtaaggg
catctaccgtaccggcgagatcatcgatctgggcgtgcaattgggcctggtcgagaagtccggcgcctggtacagctaccagggcagcaagatcg
gccagggcaaggcgaacgccgccaagtacctggaagacaatccggaaatcggttcggtactggagaagaccattcgcgaccagttgctggcca
agagcggcccggtgaaggccgacgccgaagaagtggctgacgccgaagccgattaa
triple fusion of Arabidopsis rad51 gene (SEQ ID NO: 18) 
atgacgacgatggagcagcgtagaaaccagaatgctgtccaacaacaagacgatgaagaaacccagcacggacctttccctgtcgaacagctt
caggcagcaggtattgcttctgttgatgtaaagaagcttagggatgctggtctctgtactgttgaaggtgttgcttatactccgaggaaggatctcttgca
gattaaaggaattagtgatgccaaggttgacaagattgtagaagcagcttcaaagctagttcctctggggttcacaagtgcgagccagctccatgctc
agagacaggaaattattcagattacctctggatcacgggagctcgataaagttctagaaggaggtattgaaactggttccatcacagagttatatggt
gagttccgctctggaaagactcagctgtgccatacactgtgtgtgacttgtcaacttcccatggatcaaggaggtggagagggaaaggccatgtaca
ttgatgctgagggaacattcaggccacaaagactcttacagatagctgacaggtttggattaaatggagctgatgtactagaaaacgttgcctatgcg
agggcgtataatacagatcatcagtcaaggcttttgcttgaagcagcatcaatgatgattgaaacaaggtttgctctcctgattgtcgatagtgctaccg
ctctctacagaacagatttctctggaaggggagagctttcggctcgacaaatgcatcttgcaaagttcttgagaagtcttcagaagttagcagatgagt
ttggtgtggctgttgttataacaaaccaagtagttgcgcaagtagatggttcagctcttttgctggtccccaatttaagccgattggtgggaatatcatgg
ctcatgccaccacaacaaggttggcgttgaggaaaggaagagcagaggagagaatctgtaaagtgataagctcgccatgtttgccagaagcgg
aagctcgatttcaaatatctacagaaggtgtaacagattgcaaggatgagctcggagaaggtcaaggacagggacaaggtccaggacgaggat
acgcatataagcttatgacgacgatggagcagcgtagaaaccagaatgctgtccaacaacaagacgatgaagaaacccagcacggacctttcc
ctgtcgaacagcttcaggcagcaggtattgcttctgttgatgtaaagaagcttagggatgctggtctctgtactgttgaaggtgttgcttatactccgagg
aaggatctcttgcagattaaaggaattagtgatgccaaggttgacaagattgtagaagcagcttcaaagctagttcctctggggttcacaagtgcgag
ccagctccatgctcagagacaggaaattattcagattacctctggatcacgggagctcgataaagttctagaaggaggtattgaaactggttccatca
cagagttatatggtgagttccgctctggaaagactcagctgtgccatacactgtgtgtgacttgtcaacttcccatggatcaaggaggtggagaggga
aaggccatgtacattgatgctgagggaacattcaggccacaaagactcttacagatagctgacaggtttggattaaatggagctgatgtactagaaa
acgttgcctatgcgagggcgtataatacagatcatcagtcaaggcttttgcttgaagcagcatcaatgatgattgaaacaaggtttgctctcctgattgtc
gatagtgctaccgctctctacagaacagatttctctggaaggggagagctttcggctcgacaaatgcatcttgcaaagttcttgagaagtcttcagaag
ttagcagatgagtttggtgtggctgttgttataacaaaccaagtagttgcgcaagtagatggttcagctcttttgctggtccccaatttaagccgattggtg
ggaatatcatggctcatgccaccacaacaaggttggcgttgaggaaaggaagagcagaggagagaatctgtaaagtgataagctcgccatgtttg
ccagaagcggaagctcgatttcaaatatctacagaaggtgtaacagattgcaaggatggatccggagaaggtcaaggacagggacaaggtcca
ggacgaggatacgcatatgcatgcatgacgacgatggagcagcgtagaaaccagaatgctgtccaacaacaagacgatgaagaaacccagc
acggacctttccctgtcgaacagcttcaggcagcaggtattgcttctgttgatgtaaagaagcttagggatgctggtctctgtactgttgaaggtgttgctt
atactccgaggaaggatctcttgcagattaaaggaattagtgatgccaaggttgacaagattgtagaagcagcttcaaagctagttcctctggggttc
acaagtgcgagccagctccatgctcagagacaggaaattattcagattacctctggatcacgggagctcgataaagttctagaaggaggtattgaa
actggttccatcacagagttatatggtgagttccgctctggaaagactcagctgtgccatacactgtgtgtgacttgtcaacttcccatggatcaaggag
gtggagagggaaaggccatgtacattgatgctgagggaacattcaggccacaaagactcttacagatagctgacaggtttggattaaatggagctg
atgtactagaaaacgttgcctatgcgagggcgtataatacagatcatcagtcaaggcttttgcttgaagcagcatcaatgatgattgaaacaaggtttg
ctctcctgattgtcgatagtgctaccgctctctacagaacagatttctctggaaggggagagctttcggctcgacaaatgcatcttgcaaagttcttgag
aagtcttcagaagttagcagatgagtttggtgtggctgttgttataacaaaccaagtagttgcgcaagtagatggttcagctcttttgctggtccccaattt
aagccgattggtgggaatatcatggctcatgccaccacaacaaggttggcgttgaggaaaggaagagcagaggagagaatctgtaaagtgata
agctcgccatgtttgccagaagcggaagctcgatttcaaatatctacagaaggtgtaacagattgcaaggattaactagtg
MS2-derived stem-loop for binding (SEQ ID NO: 19)
   ggccaacatgaggatcacccatgtctgcagggcc
PP7-derived stem-loop for binding (SEQ ID NO: 20)
   taaggagtttatatggaaaccctta
P22-derived stem-loop for binding B-box (SEQ ID NO: 21)
   accgccgacaacgcggt
MS2 bacteriophage coat protein (SEQ ID NO: 22) 
atggcttcaaactttactcagttcgtgctcgtggacaatggtgggacaggggatgtgacagtggctccttctaatttcgctaatggggtggcagagtgg
atcagctccaactcacggagccaggcctacaaggtgacatgcagcgtcaggcagtctagtgcccagaagagaaagtataccatcaaggtggag
gtccccaaagtggctacccagacagtgggcggagtcgaactgcctgtcgccgcttggagatcctacctgaacatggaactcactatcccaattttcg
ctaccaattctgactgtgaactcatcgtgaaggcaatgcaggggctcctcaaagacggcaatcctatcccttccgccatcgccgctaactcaggca
PP7 bacteriophage coat protein (SEQ ID NO: 23) 
atgtccaaaaccatcgttctttcggtcggcgaggctactcgcactctgactgagatccagtccaccgcagaccgtcagatcttcgaagagaaggtcg
ggcctctggtgggtcggctgcgcctcacggcttcgctccgtcaaaacggagccaagaccgcgtatcgagtcaacctaaaactggatcaggcggac
gtcgttgattgctccaccagcgtctgcggcgagcttccgaaagtgcgctacactcaggtatggtcgcacgacgtgacaatcgttgcgaatagcaccg
aggcctcgcgcaaatcgttgtacgatttgaccaagtccctcgtcgcgacctcgcaggtcgaagatcttgtcgtcaaccttgtgccgctgggccgttaa
P22 bacteriophage coat protein (SEQ ID NO: 24) 
atgacggttatcacctacgggaagtcaacgtttgcaggcaatgctaaaactcgccgtcatgagcggcgcagaaagctagccatagagcgcgaca
ccatctgcaatatcatcgattcaatttttggctgcgatgctcctgatgcttctcaggaagttaaagccaaaagaattgaccgtgtcaccaaagccatttcg
cttgccggaacgcgtcagaaggaagttgaaggaggatctgtacttcttccaggcgtagcactttacgcggctggtcatcgtaagagcaaacaaata
acagcgaggtaa
MSH2 dominant-negative allele gene sequence (SEQ ID NO: 25)

atggctgggttaaggcaggatcttagacagcatctgaagcgaatctcagatgttgagaggcttttgcgcagtctcgagagaagaagaggtgggttac
agcacattattaaactctatcaggtactttccgcacttcaatctgcttctctcaatgttaacaaaattgcattttcattgtcctaaatgtgtttatgcaactctga
agttataggtatgttattaagttcattactaattaagtcttcatcttttctctgcagtcagctataaggcttcccttcatcaaaacagctatgcaacagtacac
cggagaattcgcatcactcatcagcgagaggtacctgaaaaagcttgaggctttatcagatcaagatcaccttggaaagttcatcgatttggttgagt
gctctgtagatcttgaccagctagaaaatggagaatacatgatatcttcaaactacgacaccaaattggcatctctgaaagatcagaaagaattgct
ggagcagcagattcacgaattgcacaaaaagacagcgatagaacttgatcttcaggtcgacaaggctcttaaacttgacaaagcagcgcaatttg
ggcatgtcttcaggatcacgaagaaggaagagccaaagatcaggaagaagctgacgacacagtttatagtgctggagactcgcaaagacgga
gtgaagttcacaaacacaaagctaaaaaaactgggcgaccagtaccaaagtgttgtggatgattataggagctgtcaaaaggagctcgttgatcgt
gtagttgagactgttaccagcttctctgaggtatgtttagttattcatattaagcattggactgttacagaattggttgtttaaaatcatagtaaactatatgtg
gaatttatatgtatattgtatggttataggtatttgaggacttagctgggttactttctgaaatggatgttttgttaagctttgctgatttggctgccagttgcccta
ctccatactgtaggccagaaatcacctctttggttagtacaatctcaagttgattattttgttctgaaaatgaatagtttttctttccaagtttatgacataatgt
tgagagcacggttaataaattgtaggatgctggagatattgtactagaaggaagcagacatccatgtgtagaagctcaagattgggtgaatttcatac
caaatgattgcagactcgtaagtattgaatgtggtaaataaactgagacgtctttgtttttcttgtttcccttttgacttgaacaaatacttgtttgccctttactg
ttctttgaaatcagatgagagggaagagttggtttcaaatagtaacagggcctaacatgggagataagtccactttcatccgccaggtatgatgatttc
ctctagttcagttttgcttcatagacgtatgactaaagtcggtttccggccattataaatcccaggttggtgtgattgtgctgatggctcaagttggttcctttgt
tccttgtgataaagcatcaatttccataagagactgcatctttgcccgtgtaggagcaggcgattgccaagtgagtttaagtttagccctcaatgaacga
aaaactgctgatatcctgaacacccttattccaactttttttcctttggtgtgttagctgcgtggagtgtcaacttttatgcaagaaatgcttgaaaccgcatc
gatattgaaaggcgctactgataagtcactgataattatcgatgaacttggtcgtggaacatcaacttatgatggttttggttagtttctctgcaatttctcttc
tttcatttggatgtttttagtaagttttctattatatattcatttttatggtcatatgtgagatttcagtgctcttgacatcatcgtggtgaatatatcaggtttagcttg
ggctatatgtgagcatctggttcaagtgaaaagagcaccaactctgtttgctactcacttccatgaacttactgccttggctcaagcaaactctgaggtct
ctggtaacactgttggtgtggcaaacttccatgtcagcgctcacattgacactgaaagccgcaaactcaccatgctttacaaggtctggtttataaatta
aaaaattgctgatctgttgcagttaaaagtgtctctgtttttatgtttaatctaaattacttatttgattttcttacaaagatgaaattgaaattaattttgtgtggtg
tgttgtttgtctggttaggttgaaccaggggcctgtgaccagagctttgggattcatgtggcggaatttgccaacttccctgaaagcgtcgtggccctcgc
aagagagaaagctgcagagctggaagatttctctccctcctcgatgataatcaacaatgaggtcttgattcatttccccctttgtttttggttgatgatgga
atcattctatcattcacccattctgcagtttatgctatattattataaatctatgtgacaaagatttaattctcgtattgttgtttgcaggagagtgggaagaga
aagagcagagaagatgatccagatgaagtatcaagaggggcagagcgagctcacaagtttctgaaagagtttgcagcgatgccacttgataaaa
tggagcttaaagattcacttcaacgggtacgtgagatgaaagatgagctagagaaagatgctgcagactgccactggctcaggcagtttctgtgaa
gaacccctga

## Claims

1. A nucleic acid encoding a first fusion protein comprising an endonuclease domain and a binding domain for an origin of replication.

2. A nucleic acid according to claim 1, wherein the endonuclease cleaves a target nucleic acid molecule in a sequence specific manner.

3. A nucleic acid according to any preceding claim, wherein the endonuclease is Cas9.

4. A nucleic acid according to any preceding claim, wherein the fusion protein comprises an endonuclease and a component of the replication initiation complex or replication complex.

5. A nucleic acid encoding a second fusion protein comprising a 5' to 3' DNA exonuclease domain and an RNA binding domain.

6. A nucleic acid encoding a third fusion protein comprising a recombination inducing domain and an RNA binding domain.

7. A nucleic acid encoding a fourth fusion protein comprising a domain comprising an inhibitor of the mismatch repair pathway and an RNA binding domain.

8. A nucleic acid according to claim 5, 6 or 7, wherein the RNA binding domain binds to the RNA component of an RNA guided endonuclease for use in transformation mediated by the RNA-guided endonuclease.

9. A method of modifying the genome of a non-animal organism or cell comprising:
a. expressing in the cell the nucleic acid of any of claims 1 to 4 or introducing into the cell the first fusion protein of any of claims 1 to 4; and
b. expressing in the cell or introducing into the cell a donor nucleic acid molecule comprising an origin of replication.

10. A method according to claim 9, wherein the donor nucleic acid molecule comprises:
a. a donor nucleic acid sequence;
b. flanking nucleic acid sequences located 5' and 3' to the donor nucleic acid sequence;
c. an origin of replication 5' to the 5' flanking nucleotide sequence, and
d. a replication terminator 3' to the 3' flanking nucleotide sequence.

11. A method according to claim 9 or 10 for modifying a genome, wherein a double strand break is introduced into the genome in the presence of an exogenous donor nucleic acid molecule comprising a donor nucleic acid sequence as a template for modifying the genome or as an exogenous sequence to be integrated into the genome and a DNA repair mechanism modifies the genome via homology-directed repair (HDR).

12. A method according to claim 9, 10 or 11, further comprising the steps of:
a. expressing in the cell or introducing into the cell a sequence specific guide RNA to direct cleavage by the endonuclease domain to a specific locus; and
b. expressing in the cell one or more nucleic acids of claims 5 to 8 or introducing into the cell one or more fusions proteins of claims 5 to 8.

13. A method according to claim 12, comprising expressing in the cell two or more nucleic acids of claims 5 to 8 or introducing into the cell two or more fusion proteins of claims 5 to 8, wherein the RNA binding protein domains of the respective fusion proteins bind to different RNA sequences.

14. A fusion protein comprising an endonuclease and a component of the replication initiation complex or replication complex.

15. Use of the fusion protein of claim 14, or a nucleic acid encoding the fusion protein in transformation of a non-animal organism or cell using an RNA-guided endonuclease.
